# FASCICULE DE BREVET EUROPEEN

(11) **EP 3 512 583 B1**
(45) Date de publication et mention de la délivrance du brevet: **21.04.2021**
(21) Numéro de dépôt: 17777494.0
(22) Date de dépôt: 13.09.2017
(51) Int. Cl.: A61M 5/315

(54) **DISPOSITIF CONNECTEUR-SERINGUE POUR ADMINISTRER SÉPARÉMENT AU MOINS DEUX PRODUITS EN QUANTITÉS CONTRÔLÉES ET EN UNE SEULE INJECTION**
SPRITZENVERBINDER ZUR GETRENNTEN VERABREICHUNG VON KONTROLLIERTEN MENGEN VON MINDESTENS ZWEI PRODUKTEN IN EINER EINZIGEN INJEKTION
SYRINGE-CONNECTOR DEVICE FOR THE SEPARATE ADMINISTRATION OF CONTROLLED QUANTITIES AT LEAST TWO PRODUCTS IN A SINGLE INJECTION

(30) Priorité: 15.09.2016 FR 1658632
(43) Date de publication de la demande: 24.07.2019
(73) Titulaire: Edix SA, 1331 Luxemburg (LU)
(72) Inventeur: CHERIF-CHEIKH, Roland, 08860 Castelldefels-Barcelona (ES); BOURGOIS, Tabatha, 08025 Barcelona (ES); PARETA BELTRAN, Lluis, 08755 Castellbisball-Barcelona (ES); LACOMBE, Frederic, 08173 Sant Cugat del Valles (ES); LACHAMP, Laurence, 08850 Gava Barcelona (ES)
(74) Mandataire: Bourgouin, André
(86) Numéro de dépôt international: PCT/EP2017/073045
(87) Numéro de publication internationale: WO 2018/050709

(56) Documents cités:
- EP-A1- 0 909 155
- US-A- 4 014 330

## Description

La présente invention a pour objet un dispositif du type « connecteur-seringue ».

Par cette expression, la société demanderesse entend, dans la présente demande, définir un dispositif comprenant une partie de « type connecteur » avec au moins deux connexions proximale ou amont et distale ou aval, connections qui peuvent être reliées à des dispositifs d'injection et dispositifs comprenant une partie de « type seringue » qui comprend un ou plusieurs pistons qui peuvent être actionnés comme ceux des dispositifs d'injection par exemple manuellement par l'utilisateur.

Ce dispositif du « type connecteur-seringue » permet d'administrer séparément au moins deux produits en quantités variables par une seule aiguille laissée en place sans changer le dispositif d'injection.

Il est de plus en plus fréquent d'avoir à administrer à un patient différentes formes injectables au même moment. Il est alors souhaitable de limiter au maximum le nombre d'injections à réaliser pour le personnel soignant et le nombre de piqûres à recevoir pour le patient. Il est toutefois parfois utile, préférable, voire indispensable de séparer les produits injectés ce qui ne permet pas de les mélanger et ainsi combiner ces formes injectables en une seule injection.

Cela peut conduire à des situations dans lesquelles il faut réaliser l'une après l'autre, plusieurs injections sur une même partie du corps du malade. Il arrive qu'il soit difficile de trouver des zones de peau suffisamment saines, régénérées ou non traumatiques pour pratiquer ces injections multiples et plus particulièrement dans le cas des traitements chroniques où ces besoins d'injections se renouvellent fréquemment chaque jour et sur une période de temps prolongée.

Parmi les nombreuses utilisations possibles des dispositifs selon l'invention, on peut citer les différentes solutions injectables utilisées dans les traitements chroniques du diabète telles que les insulines rapides, semi-lentes, lentes ou basales, leurs formes pré-mélangées et les analogues du GLP-1 (Glucagon-Like Peptide 1) ou autres peptides ou molécules actives. Ces traitements peuvent être proposés en multi-doses injectables sous forme de flacons, prélevés et injectés avec des seringues type insuline ou sous forme de stylo-injecteur, injectés avec des aiguilles double-pointes à vis.

Les traitements peuvent également se présenter sous forme d'une injection pour chaque traitement, certains répétés plusieurs fois par jour, avec comme conséquence d'atteindre un nombre élevé d'injections quotidiennes. Il est connu qu'il est très important d'alterner les sites d'injection pour garder la peau saine. On sait aussi que la rapidité d'adsorption de ces injectables en sous-cutané, peut varier selon les zones d'injection dans le corps, des plus rapides, abdomen puis bras, aux plus lentes, cuisses puis fesses. Pour contrôler et reproduire les réponses thérapeutiques, il est donc aussi souhaitable d'injecter à chaque fois chaque traitement dans une même zone corporelle. Cela explique les besoins de rechercher des solutions permettant de combiner ces traitements en un nombre réduit d'injections.

Une des difficultés est que ces traitements sont souvent très personnalisés et évolutifs au cours de la vie du patient, et qu'il est alors nécessaire d'adapter les doses de chaque traitement à chaque patient et à chaque temps d'injection.

Plus généralement, on sait que les médicaments injectables ne sont pas les mieux acceptés par les patients, qui craignent les injections par peur des aiguilles et de la douleur, ni par les personnels soignants, qui connaissent les risques d'infection et les besoins d'asepsie de traitements plus compliqués et plus longs avec les injections qu'avec les médicaments oraux.

Cela entraine aussi des coûts plus élevés qui font que toutes les innovations susceptibles de simplifier les traitements injectables sont économiquement justifiées et intéressantes.

Malgré ces contraintes, le nombre des injectables augmente, entre autres parce que les nouveaux traitements biotechnologiques utilisent comme principes actifs de grosses molécules fragiles qui ne sont pas compatibles avec la voie orale, et parce que les pathologies nouvellement traitées, par exemple en endocrinologie ou en cancérologie, impliquent des traitements plus complexes, avec des produits à combiner, ainsi que plus de contraintes de précision de doses, de rapidité d'action du médicament et de problèmes de biodisponibilité.

De nombreux efforts sont faits pour essayer de réduire ces contraintes liées aux injectables qui tendent tous vers un même objectif: offrir le meilleur traitement aux malades en limitant le plus possible les inconvénients des injections, et donc logiquement, surtout pour les traitements chroniques et/ou pour la multi-thérapie, en cherchant, à l'avenir, aussi à administrer les traitements avec le moins d'injections possibles.

Cette démarche n'est pas seulement une démarche de confort pour le malade, d'économie ou de sécurité sanitaire, elle est de plus en plus souvent indispensable. Par exemple parce que la répétition et/ou la lourdeur des traitements injectables s'accompagne entre autres de problème de tolérance locale, au point qu'il devient parfois difficile pour le corps du malade de régénérer ou de cicatriser les sites lésés pour permettre des injections acceptables à la surface de sa peau.

La tolérance aux injections est particulièrement critique chez les enfants et nourrissons, plus particulièrement par voie intramusculaire. Bien que cette voie d'administration soit à éviter en pédiatrie, l'IM est parfois le seul recours de traitement possible.

C'est notamment le cas de la vitaminothérapie des nourrissons atteints de cholestase chronique, qui reçoivent tous les mois une injection de vitamines liposolubles. Cette injection est douloureuse pour l'enfant, et problématique pour les parents, qui doivent poursuivre à domicile les soins débutés à l'hôpital.

Une des solutions trouvées par le personnel de santé pour pallier cette douleur lors de l'injection est de faire précéder celle-ci par l'administration d'une dose d'anesthésique local au même site d'injection, puis de procéder à l'injection de la dose thérapeutique. L'anesthésique local peut être par exemple de la lidocaïne, de la procaïne, de la bupivacaïne, de la ropivacaïne, de l'articaïne ou de la trimécaïne.

Cette solution peut être adaptée pour tous les traitements injectables, et ce quelle que soit la zone d'injection (intradermique, sous-cutanée, intramusculaire...). Quelques médicaments injectables connus par l'homme de l'art pour être douloureux à l'injection sont, par exemple les antibiotiques, l'héparine, certains vaccins (Tetanos) ou autres produits, comme par exemple la promethazine (Phenergan®), les corticoïdes et glucocorticoïdes comme la methylprednisolone, les antianémiques, les vitamines, entre autres ; ces médicaments pourraient bénéficier de cette technique d'injection afin de réduire la douleur à l'administration.

Afin d'éviter de piquer deux fois dans la même zone, les praticiens recommandent d'injecter la vitamine par la même aiguille laissée en place, 1 à 2 minutes plus tard. La technique d'injection, qui consiste à désadapter la seringue de l'aiguille laissée en place, permet de procéder à l'administration de 2 vitamines avec une seule effraction cutanée en modifiant l'orientation de l'aiguille pour procéder à un changement de plan musculaire.

Cette technique d'injection permettant d'injecter deux produits à travers la même aiguille comporte de nombreuses incommodités, comme par exemple liées au désassemblage étanche de la connexion luer ou luer-lock aiguille-seringue, impliquant de la force et peut être difficilement fait sans effectuer des mouvements parasites qui peuvent engendrer de la douleur.

De plus, il est peu recommandable de laisser une aiguille ouverte plantée dans les tissus, qui peut créer un point d'entrée à de possibles infections. En effet, en milieu hospitalier le risque d'infections nosocomiales s'élève à 5% des patients, concerne 1 sur 20 hospitalisés, et augmente jusque 25% lorsque liés à des gestes invasifs, les germes étant transportés par les dispositifs médicaux.

Cette technique, bien que permettant l'injection de deux produits en une seule piqûre d'aiguille, n'est pas optimale et peut se réveler peu commode pour le personnel de santé comme pour le patient, en plus d'augmenter la possibilité de créer un site infectieux au niveau de la zone d'injection.

Les solutions actuelles pour réduire le nombre d'injections, qui consistent à combiner ces traitements dans une même formulation ou dans un même dispositif d'injection, n'apportent que des réponses partielles à ces besoins. Ces solutions fixent par exemple des ratios intermédiaires entre chaque traitement combiné et elles ne peuvent associer qu'un nombre très limité de produits, selon des approches qui correspondent à des moyennes plutôt qu'au traitement spécifique de chaque patient dans chaque situation.

Réduire le nombre d'injections répond aussi à d'autres besoins, par exemple dans le cas des injections d'agents thérapeutiques à usage vétérinaire où il est souvent difficile de faire accepter à l'animal plusieurs injections consécutives ou avec les animaux d'élevage pour la consommation humaine pour lesquels il est crucial de limiter les zones d'administration et les traces ou résidus d'injections. Les normes en ce sens sont très contraignantes et il est parfois difficile de trouver la bonne solution pour traiter ces animaux tout en conservant les tissus les plus sains possible sans affecter la qualité de la production.

La solution la plus simple pour réduire le nombre d'injections serait de mélanger les différents produits à injecter, mais dans la pratique se pose souvent la question de leurs compatibilités. Il est très difficile de prévoir les possibles interactions pour l'ensemble des combinaisons possibles des nombreux produits injectables. Cette approche par mélange est donc souvent risquée, elle présente des problèmes d'efficacité thérapeutique voir de tolérance des traitements ainsi combinés. Si réduire le nombre de piqûres pour le malade et donc de sites affectés pour un même traitement constitue un progrès utile face aux traitements par multi-injections, dans un grand nombre de cas cela ne peut donc souvent pas être réalisé par la solution simple du mélange de deux produits dans des proportions multiples, et encore moins de plus de deux produits, sans connaissance approfondie des conséquences de chacun de ces mélanges.

Pour limiter le nombre d'injections, il existe déjà quelques approches pour combiner au moins deux produits en une seule injection, néanmoins ces dispositifs n'apportent une solution qu'à certains des problèmes posés et qui sont résumés ici :
- pour le patient : limitation de douleur, amélioration du confort de traitement, limitation du nombre de sites d'injections surtout en traitement chronique,
- pour le personnel médical : simplicité et rapidité de la procédure d'administration, sécurité, maintien de l'asepsie, réponse face aux risques liés à l'incompatibilité du mélange des différents produits,
- pour le fabricant et les distributeurs : simplicité, rapidité d'enregistrement, adaptation aux besoins de variation des traitements, compétitivité face aux autres solutions concurrentes,
- pour les autorités d'enregistrement : sécurité sanitaire, moindre coût pour certains traitements, rapidité des soins, traitement par du personnel moins qualifié, automédication.

A la connaissance de la demanderesse, les solutions apportées à ces problèmes dans l'art antérieur peuvent être résumées comme suit :
Les premières approches existantes sont celles d'une combinaison de deux produits dans une même formulation (ci-après dénommées « combo-formulations »).

Ces options de « combo-formulations » consistent à mettre au point puis à développer comme des produits nouveaux des combinaisons très spécifiques où deux agents thérapeutiques sont formulés ensemble dans une même composition. Les incompatibilités physico-chimiques entre agents thérapeutiques, les problèmes de stabilité, les contraintes de volumes administrables par rapport aux produits séparés ou encore les besoins d'études cliniques nouvelles et complètes, limitent très fortement les avancées de ces traitements combinés. Les coûts sont souvent aussi des limites au développement industriel de telles approches.

Un exemple de telle combo-formulation à ratio fixe est le produit Xultophy® de la société NOVO NORDISK, qui est une combinaison d'insuline retard ou basale déjà commercialisée par ailleurs seule dans le produit Tresiba® et d'un analogue du GLP-1 déjà commercialisé par ailleurs seul dans le produit Victoza®. Cette combo-formulation ne peut pas couvrir tous les besoins combinés de ces deux produits, cela ne s'applique pas non plus aux patients déjà traités avec deux injections ni aux besoins de combinaisons entre insuline basale et GLP-1 analogues dans lesquels il est souhaitable de proposer au malade un ratio ou traitement personnalisé ou évolutif entre son insuline retard ou basale et son GLP-1 ou peptide analogue.

De même, la société SANOFI développe un produit comparable (Lixilan ®) de combo-formulation sous la forme de deux produits combinant l'insuline basale Lantus ® et l'analogue du GLP-1 Lyxumia®.

Un autre exemple de combo-composition est décrit dans la demande WO2014/124993. Comme pour les exemples précédents, une des limites est qu'il s'agit nécessairement d'un mélange à ratio fixe alors que chaque patient a besoin d'un traitement graduel en fonction de spécificités différentes pour les associations de traitements mises en place.

Les autres approches envisagées sont celles d'une combinaison de deux produits dans un même dispositif.

Ces options de dispositifs sont orientées vers des solutions de technologie médicale pour répondre aux mêmes besoins de combiner différents injectables en un seul produit et pour procéder à moins d'injections, sans pour cela avoir recours à de nouvelles compositions pharmaceutiques mais en utilisant des barrières physiques par le biais de dispositifs d'administrations.

L'objectif reste l'injection de plusieurs produits avec une seule implantation d'aiguille, sans mélanger les fluides ou liquides à injecter et/ou en réduisant autant que possible leur contact pour obtenir des effets comparables à ceux de leurs injections séparées. Ici aussi il s'agit le plus souvent, en pratique, de combiner seulement deux produits.

On distingue en général deux approches principales pour répondre à ce problème : soit les produits sont placés dans deux réservoirs en Y ou en parallèle, soit, ils sont placés dans des réservoirs en ligne ou l'un derrière l'autre.

La première solution consiste par exemple à placer deux produits en parallèle l'un à côté de l'autre, le plus souvent dans deux réservoirs séparés qui débouchent dans une même connexion en Y. Les réservoirs peuvent être actionnés par un même système ou par deux systèmes de pression ou transfert distincts. Ces dispositifs sont encombrants, plus volumineux que les dispositifs d'injections classiques. Ils permettent aux produits de rester séparés, mais ils n'éliminent pas les risques de contaminations croisées ou les possibles incompatibilités chimiques.

De même, il est compliqué dans ces cas de ne pas injecter les deux produits selon un ratio dose/volume fixe.

Au moment de l'administration, il reste difficile d'éviter que les deux produits ne se mélangent dans la connexion vers l'aiguille unique, tout au long des canaux communs d'injection, selon un processus agité ou dynamique, ce qui augmente les risques de problèmes de compatibilité entre les deux produits et limite les applications en fonction des types de produits et de leurs compatibilités sous formes liquides injectables.

Un dispositif d'injection par une seule aiguille de liquides arrangés dans un montage de réservoirs « en parallèle » est décrit dans le brevet US4609371 ; ce système présente l'inconvénient majeur d'être plus large que les dispositifs d'injections classiques.

Pour chercher à répondre aux limites posées par le fonctionnement en parallèle des 2 pistons la demande WO2012/072559 propose un dispositif dans lequel ces déplacements sont commandés par des moteurs indépendants contrôlés par un dispositif électronique, permettant de doser à volonté les volumes injectés de chaque produit. Cela conduit à un ensemble complexe lourd, cher et dépendant de piles ou batteries.

Un exemple d'application d'un tel dispositif combine l'insuline Glargine retard ou basale déjà commercialisée par ailleurs seule dans le produit Lantus® et un peptide analogue du GLP-1 déjà commercialisé par ailleurs seul dans le produit Lyxumia®. Dans ce cas aussi, il s'agit d'un ratio fixe d'injection entre les deux produits.

La seconde solution consiste à placer dans un dispositif les deux produits en ligne, l'un derrière l'autre. Ces dispositifs sont moins encombrants ou volumineux que les systèmes en parallèle, mais les produits sont moins facilement, ou moins efficacement séparés ou isolés dans la phase de stockage et dans la phase d'injection. Ils sont donc soumis à plus de risques de contamination croisée. Ils doivent souvent être remplis dans des réservoirs moins classiques, selon des méthodes plus spécifiques, moins validées, moins universelles et donc plus chères.

Il est compliqué aussi, voire impossible dans ces cas de ne pas injecter les 2 produits selon un ratio dose ou volume fixe. Cela ne permet donc pas de moduler leurs doses en fonction des besoins.

Dans le brevet USP 3911916 est décrit un dispositif dans lequel une aiguille double pointe perce les pistons en fin d'injection et permet ainsi de mettre séquentiellement les compartiments (ou chambres) placés en aval en contact avec l'aiguille d'injection. On peut aussi citer le brevet USP 3.923,058 qui décrit un dispositif similaire.

Plus récemment ont été développés des dispositifs autour de seringues bi-compartimentales avec dans la plupart des cas un by-pass pratiqué dans le cylindre. Ces conditionnements sont chers et complexes et peuvent poser des problèmes d'étanchéité ou de stabilité spécifiques. Comme exemples de telles présentations on peut citer les dispositifs décrits dans les brevets US5080649, US2011092906 ou US2014103045.

D'autres approches ont été proposées au problème du by-pass à partir d'un seul réservoir segmenté par des pistons comme dans le cas du brevet USP 4.439.184.

Dans le brevet USP 4723937 le dispositif comporte un seul réservoir et un piston intermédiaire : la partie distale (ou avale) est vidée par ce piston qui vient se positionner dans une pièce qui fait office de by-pass en déformant le piston pour vider la partie proximale (ou amont).

Dans les brevets USP 3680558, et 4702737 le dispositif utilise un système avec deux ou plusieurs réservoirs qui circulent les uns dans les autres. Cette solution implique des réservoirs différents et spécifiques, s'affinant depuis l'aval vers l'amont, donc nécessairement de moindre volume, ce qui peut obliger à rallonger l'ensemble jusqu'à obtenir rapidement une longueur incompatible avec la distance habituelle du geste d'injection. Ces dispositifs spécifiques comportent des doses fixes et des présentations pré-remplies.

Dans les demandes WO 0126718 et WO 9746202 est décrit un dispositif dans lequel un réservoir d'injection proximal ou amont, telle qu'une cartouche, peut être utilisé comme une tige sur un piston indépendant qui porte un système de connexion à ce réservoir amont pour collapser et donc pour vider le contenu d'un réservoir d'injection distal ou aval, telle qu'une seringue, au cours de l'injection du contenu de celui-ci à travers une aiguille. Cette approche a été reprise dans la demande US2014/0276039.

Dans la demande US 2013/0267932 le réservoir amont peut être une cartouche, dans ce cas précis actionnée par un stylo-injecteur. Cette solution présente des risques de contamination croisée, avec possibilité que le premier produit aval entre en contact avec le second produit amont dans la cartouche multi-doses Un autre inconvénient est que ce premier produit aval injecté reste de type uni-dose, pré-rempli à dose fixe.

Dans la demande de brevet US 2010/0286513 on trouve aussi cités d'autres dispositifs qui proposent des solutions pour délivrer différents injectables par une seule piqûre, soit avec 2 seringues en parallèle, soit en ligne avec des réservoirs télescopiques. Dans le brevet US 3680558 est décrit un « telescoping compartments » comportant une valve s'ouvrant et se fermant sous l'effet de la rotation du piston, remplissable, mais ce dispositif est complexe et imprécis.

Le brevet américain US 4.014.330 décrit une seringue à usage unique du type pré-remplie comportant deux compartiments.

Le brevet européen EP 0.909.155 décrit un procédé pour constituer une préparation injectable dans lequel on introduit un liquide dans un volume de forme sèche d'un principe actif conditionné sous vide.

Cependant les dispositifs décrits dans ces brevets ne permettent pas d'administrer séparément au moins deux produits en quantités contrôlées par le biais d'une seule injection.

Le dispositif connecteur-seringue selon l'invention offre une réponse nouvelle à tous ces besoins. Il permet de combiner tous ces traitements à toutes les doses ou tous les ratios selon le cas spécifique de chaque patient à chaque instant de l'évolution de sa maladie.

La demanderesse a inventé des dispositifs d'un nouveau type « connecteur-seringues » tels que définis ci-dessus qui apportent des solutions nouvelles et d'application générale à l'ensemble des besoins existants pour limiter les injections dans tous les traitements qui nécessitent plusieurs produits injectables.

Comme indiqué ci-dessus, même s'il existe quelques solutions pour répondre à ces besoins, elles ne permettent en générale de combiner que deux produits précis dans des systèmes pré-remplis et/ou d'application spécifiques. Elles n'ouvrent pas le champ des applications et combinaisons possibles et ne sont que des solutions ponctuelles face à une problématique beaucoup plus générale non résolue.

### DEFINITIONS :

On utilisera dans l'ensemble de la présente demande les définitions suivantes :
Par réservoir s'entend un volume contenu dans le dispositif susceptible de contenir un produit, de préférence de forme cylindrique.
Par joint s'entend un élément souple compressible susceptible de faire le contact de manière hermétique entre 2 parties rigides, de préférence un anneau circulaire en caoutchouc butyle.
Par embout luer ou luer-lock s'entend l'extrémité mâle ou femelle standard de seringue de forme tronconique sur laquelle vient se fixer par contact l'extrémité femelle standard de l'aiguille.
Par adaptateur stylo-injecteur s'entend une pièce plastique spécifique et amovible qui transforme l'aiguille d'injection du dispositif selon l'invention en l'équivalent de l'aiguille interne d'une aiguille à stylo-injecteur. Cette pièce sert de leurre pour visser le stylo-injecteur sur l'une des extrémités du dispositif selon l'invention, ainsi que de protection pour cacher l'aiguille d'injection jusqu'au moment de l'administration.
Par solution universelle/standard sont définis les produits qui peuvent être administrés et sont contenus dans des contenants standards et connus par l'homme de l'art tels que les flacons, les poches, les ampoules, les cartouches, les bouteilles, les seringues et les stylos-injecteurs. Il n'est pas nécessaire de les modifier pour pouvoir les administrer avec le dispositif réservoir selon l'invention
Par piston s'entend la partie mobile à l'intérieur du corps du dispositif ou réservoir séparant deux produits. Il permet de vider en collapsant de manière séquentielle le contenu de la chambre du réservoir qui se trouve en aval.
Par creux s'entend d'une pièce percée d'un canal de connexion ouvert en permanence sans besoin de mécanisme de fermeture-ouverture, permettant le passage de produits de la partie proximale à la partie distale.
Par administration séquentielle, s'entend, administrations effectuées l'une après l'autre mais de façon continue, sans qu'il existe un contact entre les produits durant le processus.
Par distale s'entend la zone du dispositif la plus proche de la zone d'administration. Peut aussi se définir comme la partie en aval du dispositif en prenant comme référence le déplacement du fluide à travers un dispositif.
Par proximale s'entend la zone du dispositif la plus éloignée de la zone d'injection. Peut aussi se définir comme la partie en amont du dispositif en prenant comme référence le déplacement du fluide à travers un dispositif.
Par produit s'entend sans limitation et à titre d'exemples, les substances, agents ou préparations thérapeutiques ou prophylactiques sous forme de formulations injectables, liquides, solides ou semi-solides ou encore fluides, pâteuses ou gels. De manière préférentielle, les produits sont des liquides ou des fluides en général.
Par injection s'entend le passage des produits l'un après l'autre par le même canal pour aboutir dans la même zone. Cette terminologie peut donc aussi s'appliquer à d'autres situations telles que, par exemple l'administration d'agents thérapeutiques ou prophylactiques par le biais d'une aiguille, les administrations topiques ou ophtalmiques ou dans tous autres domaines d'application.

On entend par connexions sans limitation et à titre d'exemples, les connexions standard ou embouts universels mâles ou femelles, luer, luer-lock, vis, septum, aiguille

Les descriptions qui suivent ne constituent que quelques exemples avantageux de modes de réalisation du dispositif selon l'invention, lequel pourra être mis en œuvre selon d'autres spécificités également couvertes par l'invention.

Les dispositifs et procédés de la présente invention utilisent des arrangements en ligne mais contrairement à certaines des solutions précédentes, les dispositifs connecteur-seringues selon l'invention peuvent être non pré-remplis, ils s'adaptent à toutes les doses/volumes, à toutes les combinaisons de dispositifs d'injection de toutes les présentations injectables standards et ils s'utilisent selon des procédés existants de préparations extemporanés.

Comme indiqué ci-dessus, les solutions en ligne qui utilisent l'approche d'un réservoir amont qui entre dans le réservoir aval conditionnent et limitent les formes et volumes de chacun des réservoirs d'injection et augmentent la longueur de l'ensemble. Cela limite aussi la précision des dosages qui sont réalisés par ces réservoirs par rapport aux dispositifs selon la présente invention dans laquelle les diamètres du piston creux interne sont indépendants des volumes des réservoirs des dispositifs d'injection connectés et des dosages, et leur précision sont assurés par ces réservoirs indépendants ce qui permet de réduire la longueur du dispositif, similaire à celle d'un système d'injection standard.

La présente invention qui comporte un piston interne toujours ouvert est aussi une solution beaucoup plus simple que, par exemple, les systèmes à valve ou à septum et est indépendante des caractéristiques des produits à combiner pour le remplissage comme pour l'injection.

De façon surprenante, la demanderesse a maintenant découvert le moyen d'éviter des solutions complexes, chères et longues à développer pour obtenir avec des dispositifs constitués d'un nombre restreint de pièces basiques, des avantages de simplicité et de confort d'utilisation, tout en permettant toutes les combinaisons possibles avec les outils et les gestes préparatoires standards de remplissage-préparations, de contrôle, d'amorçage et d'injection.

### BREVE DESCRIPTION DES FIGURES

Figures 1 à 9 :
La figure 1 représente un dispositif connecteur-seringue dans un mode d'exécution comprenant sur le piston creux, une connexion femelle luer (ou luer-lock) (1a) et sur le réservoir, une connexion mâle luer-lock (2a).
La figure 2 représente un dispositif connecteur-seringue dans un mode d'exécution comprenant sur le piston creux, une connexion femelle luer (ou luer-lock) (1a) et sur le réservoir, une aiguille fixe (4).
La figure 3 représente le dispositif connecteur-seringue dans un mode d'exécution semblable à celui de la figure 2 dans lequel on ajoute sur l'aiguille fixe du réservoir, une connexion séparée adaptateur stylo-injecteur (11).
La figure 4 représente le dispositif connecteur-seringue dans un mode d'exécution comprenant un piston plein (12) et un piston creux (1).
La figure 5 représente des détails du réservoir et du piston plein dans sa position après utilisation du dispositif connecteur-seringue.
La figure 6 représente un dispositif connecteur-seringue selon la version de la figure 4 avec un adaptateur stylo-injecteur dans un procédé d'association à deux stylo-injecteurs.
La figure 7 représente un dispositif connecteur-seringue selon la version de la figure 2 avec un bouchon luer (8) sur sa connexion proximale, dans son procédé d'association à un flacon de produit injectable (9).
La figure 8 représente un dispositif connecteur-seringue selon la version de la figure 2 avec un bouchon sur l'aiguille de sa connexion distale, dans son procédé d'association à deux seringues de produits injectables.
La figure 9 représente le dispositif connecteur-seringue dans une version avec un mécanisme de blocage après utilisation et de profondeur de l'aiguille.

Un premier objet de la présente invention est un dispositif du type connecteur-seringue pour administrer séparément au moins deux produits en quantités contrôlées par le biais d'une seule injection, comprenant-un piston creux (1) pouvant être déplacé à l'intérieur d'un réservoir (2) caractérisé en ce que :
- ledit piston creux (1) est ouvert par un canal interne (1b), de l'extrémité introduite à l'intérieur dudit réservoir (2) à l'autre extrémité dans une connexion proximale (1a)
- ledit piston creux (1) forme une barrière étanche avec la paroi interne (2b) dudit réservoir (2) et dans sa course fait varier le ou les volumes dudit réservoir (2) selon sa position d'une extrémité à l'autre à l'intérieur dudit réservoir (2) ;
- ladite connexion proximale (1a) reste constamment accessible à l'extérieur dudit réservoir (2) et
- ledit réservoir est ouvert à une extrémité dans une connexion distale (2a) et à l'autre extrémité de façon à introduire et laisser circuler ledit piston creux (1).

Les dispositifs selon l'invention peuvent être à usage unique à un coût très intéressant comparé aux améliorations de traitements qu'ils permettent. Ces dispositifs fonctionnent manuellement selon les mêmes principes que les dispositifs connecteurs et seringues actuels, et ne requièrent aucune assistance, ni moteur, ni piles ou batteries.

Les produits sont préférentiellement administrés de façon séquentielle, dans l'ordre souhaité, à des vitesses et selon des temps contrôlés et avec de possibles variations de sites ou de profondeurs d'administration à partir d'un même point initial d'injection.

Les dispositifs type connecteur-seringue selon l'invention pourront être réalisés en différentes tailles et avec différents types de connexions proximales et distales. Ils proposent une solution universelle pour toutes les administrations combinées ou les co-traitements injectables qui posent problème, indépendamment des produits à administrer et de leurs contraintes spécifiques ou du domaine d'application. Ils peuvent s'adapter à tous les produits à administrer tels que, à titre d'exemple, tous les injectables liquides sous formes de solutions ou de suspensions, préalablement lyophilisés ou non.

Comme indiqué ci-dessus, l'invention propose un dispositif pour l'injection unique d'au moins deux produits liquides, solides ou semi-solides, qui à la fois permet d'éviter tout contact entre les produits, peut permettre d'injecter une gamme étendue de volumes susceptibles de s'adapter à tous les traitements, peut utiliser tous les dispositifs d'injections actuels tels que aiguilles, seringues, stylo-injecteurs, cartouches ou systèmes de perfusion flacons poches ou autres et peut être simple, économique à fabriquer et d'un encombrement réduit compatible avec ces dispositifs.

En particulier, le dispositif selon l'invention pour l'injection d'au moins deux produits tels que par exemple deux liquides thérapeutiques, dans les tissus d'un patient, comprend au moins un piston creux qui délimite au moins une chambre intérieure à volume variable apte à contenir au moins un premier produit dans un réservoir ;
ledit piston creux dans ledit réservoir est toujours ouvert par un canal interne sous forme d'un conduit intérieur reliant son extrémité avale à son extrémité amont dans une connexion proximale, ledit conduit débouchant de cette manière vers l'extérieur du dispositif ;
ledit réservoir est ouvert également à son extrémité avale dans une connexion distale qui peut porter un élément d'injection telle qu'une aiguille ;
ledit piston creux est mobile coulissant dans ledit réservoir de manière étanche et est suffisamment long pour que son extrémité amont sous forme de connexion proximale qui peut porter un dispositif d'injection telle qu'une seringue, reste accessible à l'extérieur du corps réservoir même lorsque le piston vient collapser le fond du réservoir en aval.

L'extrémité amont dudit piston creux est susceptible d'être actionnée et/ou saisie par l'utilisateur/trice pour le déplacer en translation.

Ledit piston creux peut jouer le rôle de tige de piston et collapser seul le réservoir, sans que ledit dispositif d'injection ait à entrer dans ledit réservoir.

Ledit piston creux permet de relier la sortie dudit réservoir qui peut porter une aiguille, à une autre source de produit dans un autre dispositif d'injection aval et indépendante dudit dispositif.

La connexion proximale dudit piston creux pourra être un des adaptateurs qui permettent son branchement direct aux systèmes d'injection classique tels que par exemple et sans limitations, luer, luer-lock, vis, septum ou aiguille.

Ce dispositif est compatible avec toutes les tailles de seringues et tous les types de réservoirs d'injection et avec une utilisation possible non pré-rempli par remplissages en extemporané de deux ou plusieurs produits.

Le passage des produits, de préférence liquides, dans le canal toujours ouvert du piston creux peut se faire dans les deux sens. Le remplissage du réservoir peut donc se faire par les deux extrémités ce qui permet tous types d'arrangements et de chargements.

Dans une version particulière du dispositif connecteur-seringue selon l'invention, on ajoute un piston plein dans le réservoir. Ce piston plein est indépendant du piston creux, il va isoler les volumes avals des volumes amont du réservoir et ne mettre ceux-ci en communication avec la sortie distale du réservoir par une modification de la surface dudit réservoir que lorsque ledit piston plein se trouve au fond dudit réservoir.

La présente invention a plus particulièrement pour objet un dispositif tel que décrit ci-dessus et comprenant au moins un, de préférence un seul piston plein (12) pouvant se déplacer à l'intérieur dudit réservoir (2) caractérisé en ce que :
- le au moins dit piston plein (12) est introduit dans ledit réservoir (2) avant l'introduction dudit piston creux (1) et
- ladite paroi interne (2b) présente à partir dudit fond (2c) dudit réservoir (2), de préférence sur une distance pratiquement égale à l'épaisseur dudit piston plein une variation de sa section destinée à rompre l'étanchéité entre ledit piston plein (12) et ladite paroi interne (2b) dudit réservoir (2), de préférence une augmentation de son diamètre (18a)
- A l'exception de la distance sur laquelle ladite paroi interne (2b) présente une variation de sa section (18a), ledit piston plein (12) forme une barrière étanche avec ladite paroi interne (2b) dudit réservoir (2)
- lorsque ledit piston plein (12) se trouve au contact dudit fond (2c) dudit réservoir (2) il n'est plus étanche avec ladite paroi interne (2b) dudit réservoir (2) ; ce qui permet le passage desdits produits entre ledit piston plein (12) et ladite paroi interne (2b) dudit réservoir (2),

La présente invention a plus particulièrement pour objet un dispositif tel que décrit ci-dessus et comprenant un seul piston plein (12).

Dans un mode d'exécution de l'invention, pour permettre le passage du volume du ou des produits situés en amont du piston plein (12) on peut réaliser une variation de surface du au moins un piston plein, par exemple sous la forme d'au moins une gorge ou une rainure.

La présente invention a donc pour objet un dispositif comprenant au moins un piston plein (12), de préférence un seul piston plein, caractérisé en ce que le au moins piston plein comporte sur sa face distale une variation de surface de préférence au moins une gorge ou une rainure (18b).

Dans un mode d'exécution de l'invention, la paroi interne (2b) présente à partir dudit fond (2c) dudit réservoir (2), de préférence sur une distance pratiquement égale ou légèrement supérieure à l'épaisseur dudit piston plein une variation de sa section destinée à rompre l'étanchéité entre ledit piston plein (12) et ladite paroi interne (2b) dudit réservoir (2), de préférence une augmentation du diamètre de la section (18a)

Lorsque la variation de la section du réservoir consiste en une augmentation du diamètre de ladite section, l'expression augmentation de diamètre (18a) signifie qu'en pratique le diamètre de la paroi interne dudit réservoir pourra présenter une augmentation sur une certaine longueur. Cette augmentation de diamètre pourra être supérieure de 1% à 50% par rapport au diamètre du piston plein.

La longueur limitée sur laquelle est réalisée cette augmentation de diamètre (de la paroi interne du réservoir) est aussi du même rang de valeurs/pourcentage par rapport à l'épaisseur dudit réservoir, et sur le reste de la longueur dudit réservoir le diamètre du piston et celui de la paroi interne dudit réservoir sont pratiquement identiques car le piston plein forme une barrière étanche avec ladite paroi interne.

La longueur de la partie du réservoir présentant une augmentation de surface pourra être supérieure de 5% à 60% par rapport à l'épaisseur du piston plein.

De manière préférentielle, le diamètre dudit réservoir pourra être augmenté de 1,5% à 30% par rapport au diamètre du piston sur une longueur qui pourra être supérieure de 10% à 50% par rapport à l'épaisseur du piston.

De manière encore plus préférentielle, le diamètre dudit réservoir pourra être augmenté de 1.75% à 20% par rapport au diamètre du piston sur une longueur qui pourra être supérieure de 15% à 40% par rapport à l'épaisseur du piston.

De manière préférée, le diamètre dudit réservoir pourra être augmenté de 2% à 10% par rapport au diamètre du piston sur une longueur qui pourra être supérieure de 20% à 30% par rapport à l'épaisseur du piston.

A titre d'exemple illustratif et non limitatif de la présente invention, les inventeurs ont construit un prototype selon l'invention qui a été utilisé pour injecter deux produits liquides. Dans ce prototype, le diamètre du piston plein est de 8,2 mm et son épaisseur est de 2,75mm.

Dans le dispositif réalisé selon l'invention, le diamètre dudit réservoir a été augmenté de 2.44% par rapport au diamètre du piston ce qui conduit à un diamètre du réservoir de D=8.4 mm sur une longueur supérieure de 27.27% par rapport à l'épaisseur du piston ce qui conduit à une longueur de L=3.5mm.

L'expression pratiquement égale ou légèrement supérieure au diamètre du piston signifie également que de préférence, l'espace entre la paroi interne et le piston au niveau de l'augmentation de diamètre est toujours égale à au moins 0,2mm. En d'autres termes, à ce niveau du réservoir, le diamètre de sa paroi interne doit être supérieur d'au moins 0,2 mm au diamètre du piston.

L'expression pratiquement égale ou légèrement supérieure à l'épaisseur du piston signifie également que de préférence l'augmentation de diamètre du réservoir est toujours réalisée sur une distance égale à l'épaisseur du piston augmentée d'au moins 0,5mm.

Dans un mode préféré, cette augmentation de diamètre sur une distance pratiquement égale à l'épaisseur du piston plein est telle que lorsque le piston plein se trouve dans cette position, un espace est ainsi créé entre la paroi du réservoir et le tour du piston plein, espace qui peut être équivalent à la surface interne des aiguilles ou du canal interne, de façon à ne pas modifier à ce niveau les forces nécessaires pour déplacer les fluides chargés.

Ainsi, le dispositif selon l'invention, comprend au moins un, de préférence un seul piston plein (12) pouvant se déplacer à l'intérieur dudit réservoir (2) caractérisé en ce que :
- le au moins dit piston plein (12) est introduit dans ledit réservoir (2) avant l'introduction dudit piston creux (1) et
- ladite paroi interne (2b) présente à partir dudit fond (2c) dudit réservoir (2), de préférence sur une distance pratiquement égale à l'épaisseur dudit piston plein une augmentation de surface ou de diamètre (18a)
- A l'exception de la distance sur laquelle ladite paroi interne (2b) présente une variation de surface ou de diamètre (18a), ledit piston plein (12) forme une barrière étanche avec ladite paroi interne (2b) dudit réservoir (2)
- lorsque ledit piston plein (12) se trouve au contact dudit fond (2c) dudit réservoir (2) il n'est plus étanche avec ladite paroi interne (2b) dudit réservoir (2) ; ce qui permet le passage desdits produits entre ledit piston plein (12) et ladite paroi interne (2b) dudit réservoir (2).

La présente invention peut par exemple reprendre l'approche décrite ci-dessus qui cherche à combiner un produit existant pré-rempli en stylo-injecteur mais avec l'avantage de pouvoir le combiner avec d'autres produits pré-rempli existants tel qu'un autre stylo-injecteur tout en faisant varier les doses de chaque produit comme on le souhaite pour les ajuster à chaque patient selon son traitement spécifique, en accord notamment avec les orientations actuelles de la médecine personnalisée.

La présente invention a plus particulièrement pour objet un dispositif tel que décrit ci-dessus, caractérisé en ce que ledit connecteur-seringue peut être connecté à un ou plusieurs dispositifs d'administration du type seringue, stylo-injecteur, poche d'injection, flacon, injecteur, pompe, aiguille, cathéter ou connecteur.

Ainsi, l'utilisateur peut selon ses besoins connecter au dispositif selon l'invention par exemple des seringues et/ou des stylo-injecteurs et/ou des systèmes de perfusion dans leur configuration commerciale sans aucun besoin de manipulation ni développement additionnel.

Il peut décharger de façon séquentielle dans le dispositif de l'invention les agents thérapeutiques ou prophylactiques sans manipulation et procéder à leur injection jointe selon des méthodes standards.

Le dispositif selon l'invention peut ne pas être un dispositif chargé dont le ou les réservoirs devraient, ensemble ou séparément, être enregistrés comme autant de produits spécifiques. Comme il peut ne pas être un système pré-rempli, il ne doit pas nécessairement solutionner les problèmes potentiels de stabilité, compatibilité et d'étanchéité durant leur cycle de vie de certains dispositifs préalablement cités.

En utilisant le dispositif selon l'invention, le patient peut recevoir au moins deux agents thérapeutiques en une seule injection à des volumes différents et ce, quelle que soit la présentation dans laquelle se trouve les agents thérapeutiques injectables.

Les dispositifs connecteur-seringue selon l'invention sont préférentiellement arrangés en ligne de façon à pouvoir être utilisés comme des connecteurs avec d'autres dispositifs d'injection à chacune de leurs extrémités, telle qu'une aiguille connectée à l'extrémité distale ou avale et une seringue connectée à l'autre extrémité proximale ou amont et de façon à pouvoir ensuite être utilisés comme des seringues au moment de l'injection.

La présente invention a plus particulièrement pour objet un dispositif tel que décrit ci-dessus, caractérisé en ce que le diamètre dudit canal interne (1b) est le plus petit possible qui soit compatible avec le passage desdits produit à administrer et de préférence ledit diamètre est d'un diamètre supérieur ou au moins égal au diamètre du conduit de l'aiguille (4) lorsque la connexion distale (2a) est pourvue de ladite aiguille. Par exemple, pour injecter deux produits liquides (de volumes compris par exemple entre 5µL et 2mL), le dispositif pourra être équipé d'une aiguille pouvant aller de gauge 25 (soit 0,5mm de diamètre extérieur et 0,3mm de diamètre interne) et d'un piston percé d'un canal interne de diamètre minimal égal à 0,3mm, préférentiellement de 0,4mm de diamètre. Pour deux produits liquides, on utilisera préférentiellement une aiguille de diamètre réduit, par exemple de gauge 29 (soit 0.34mm de diamètre extérieur et 0,18mm de diamètre interne) et un piston percé d'un canal interne de diamètre minimal égal à 0,18mm. Pour l'injection de produits visqueux, le dispositif pourra être équipé par exemple d'une aiguille égale ou inférieure à 23G (soit 0,64mm de diamètre extérieur et 0,34mm de diamètre interne) avec un piston percé d'un canal interne de diamètre égal ou supérieur à 0,34mm, ou même d'une aiguille de 18G (soit 1,2mm de diamètre externe et 0,8mm de diamètre interne) avec un piston percé d'un canal interne de diamètre égal ou supérieur à 0,8mm.

Dans ce qui suit, on entend par connexions sans limitation et à titre d'exemples, les connexions standard ou embouts universels mâles ou femelles, luer, luer-lock, vis, septum, aiguille. On entend par dispositifs d'injections sans limitation et à titre d'exemples, les aiguilles, les cathéters, les connecteurs, les seringues, les stylo-injecteurs, les systèmes de perfusion flacons, poches ou les pompes. On entend par produit sans limitation et à titre d'exemples, les substances, agents ou préparations thérapeutiques sous forme de formulations injectables, liquides, solides ou semi-solides ou encore fluides, pâteuses ou gels.

Les connexions des dispositifs selon l'invention sont préférentiellement standards telles que luer, luer-lock ou à vis-aiguille de façon à en faire des adaptateurs universels sur les dispositifs d'injections tels que seringues, stylo-injecteurs ou dispositifs de perfusion.

La présente invention a encore plus particulièrement pour objet un dispositif tel que décrit ci-dessus, caractérisé en ce que ledit connecteur-seringue comporte à ses extrémités proximale et/ou distale des connexions (1a) et (2a) qui peuvent être mâles ou femelles, et être choisies parmi les types luer, luer-lock, vis, aiguille, septum, valve ou un dispositif caractérisé en ce qu'un adaptateur (11) qui permet la fixation d'un stylo-injecteur audit connecteur-seringue est fixé sur ladite connexion distale (2a) autour de son aiguille (4).

Les dispositifs connecteur-seringues selon l'invention peuvent permettre de pratiquer cette injection unique dans les différentes approches classiques des injections telles que par exemple pour les injections intradermiques, sous-cutanées, intramusculaires ou intraveineuses. Cette injection combinée peut être réalisée exactement au même site par exemple à la sortie du point fixe d'injection dans l'un quelconque de ces tissus. Ces injections combinées peuvent aussi être réalisées en des points différents. Selon l'arrangement retenu en fonction des forces d'implantation de l'aiguille et de collapse dudit dispositif, on pourra par exemple commencer l'injection sous-cutanée du produit le plus aval dès l'introduction de l'aiguille sous la peau et poursuivre cette injection jusqu'à la terminer avant d'arriver au point de pénétration le plus profond de l'aiguille où il sera possible de pratiquer dans une autre zone l'injection d'un autre produit. On pourra également réaliser cette séparation en des points fixes avec un arrangement tel que décrit ci-après. Il est également possible selon le mode séquentiel de ces injections de laisser un lapse de temps contrôlé entre chaque injection si cela favorise l'efficacité des traitements.

Les dispositifs connecteur-seringues selon l'invention permettent des dosages précis pour chacun des produits ou constituants de l'injection combinées qui ne dépendent que des dispositifs d'injection auxquels ces connecteur-seringues sont connectés et qui sont donc équivalents à ceux obtenus dans l'utilisation habituelle de ces dispositifs d'injection. Ces dispositifs connecteur-seringues permettent donc aussi des doses réglables et variables pour chacun des constituants injectés ensemble.

Les dispositifs connecteurs seringues selon l'invention sont préférentiellement à usage unique, ils sont adaptables à tous les besoins des thérapies injectables. Ils sont préférentiellement remplis extemporanément juste avant leur utilisation mais ils peuvent aussi être pré-remplis de l'un ou de l'ensemble des produits qu'ils doivent injecter.

Les dispositifs selon l'invention sont adaptés aux différentes formes galéniques injectables telles que liquides, semi-solides ou solides par exemples lyophilisées ou en poudres comme par exemple les formes retard micro-particulaires. On pourra par exemple n'avoir qu'un seul produit injecté pré-rempli sous forme solide lyophilisée et connecter le dispositif au réservoir liquide qui ne contient que son mélange de reconstitution.

Les dispositifs connecteur-seringues selon l'invention peuvent selon leur taille et leur application, s'adapter à tous les volumes d'injections, par exemple depuis quelques dizaines de microlitres pour certaines applications comme le diabète ou en vétérinaire sur de petits animaux, jusqu'à quelques centaines de millilitres, voir quelques litres par exemple pour les perfusions ou pour certaines applications vétérinaires sur de gros animaux.

Les dispositifs connecteur-seringues selon l'invention permettent d'injecter un seul produit. Ils permettent de combiner en une seule injection l'administration d'au moins deux produits voir de trois produits maintenus séparément. Ils permettent aussi d'injecter plus de trois produits en une seule injection.

Chacun des volumes d'injection définis par les dispositifs connecteur-seringues selon l'invention peut contenir zéro, un ou plusieurs produits à injecter ou encore un excipient qui va constituer un produit à injecter avec le contenu d'un autre volume qui pourra par exemple être sous forme solide. Ces dispositifs et leurs volumes peuvent aussi contenir toute autre substance qui nécessiterait d'être séparée jusqu'au moment et au point où l'on souhaite les réunir. Par injection on entend aussi des produits qui passent l'un après l'autre par le même canal pour aboutir dans la même zone, cette terminologie peut donc aussi s'appliquer à d'autres situations telles que par exemple dans l'administration d'agents thérapeutiques ou prophylactiques, les administrations topiques ou ophtalmiques ou dans tous autres domaines d'application.

On peut associer aux dispositifs selon l'invention les systèmes de protections classiques telles que par exemple des protections d'aiguilles.

La présente invention a encore plus particulièrement pour objet un dispositif tel que décrit ci-dessus caractérisé en ce que ledit connecteur-seringue est stérilisé avant remplissage et est réalisé en matériaux compatibles avec les injections, en ce que ledit réservoir est transparent et en ce que ledit piston creux ou ledit réservoir peuvent être gradués.

La présente invention a également plus particulièrement pour objet un dispositif tel que décrit ci-dessus, caractérisé en ce qu'une partie de la connexion distale pourvue d'une aiguille est prolongée sur un diamètre comparable à celui dudit dispositif, jusqu'à la canule de ladite seule aiguille pour assurer un appui sur la peau ou la zone d'administration au moment de l'injection.

La présente invention a également plus particulièrement pour objet un dispositif tel que décrit ci-dessus caractérisé en ce que ledit connecteur-seringue présente un mécanisme de blocage qui évite sa réutilisation et qui peut contrôler différentes profondeurs d'injection de ladite seule aiguille lorsque ledit piston creux est introduit audit fond dudit réservoir.

Le dispositif selon l'invention peut être équipé d'un système ou mécanisme de sécurité, qui, une fois l'injection terminée recouvre l'aiguille (shield needle), ou d'un système d'aiguille rétractable pour diminuer les risques de blessures par l'utilisateur (dispositif plus sûr) et assurer son usage unique (impossible de réutiliser le dispositif une fois le système activé).

Les dispositifs connecteur-seringue selon l'invention pourront être déclinés dans toutes les tailles compatibles avec chacune de leurs applications possibles et par exemple dans le domaine thérapeutique injectables permettre des volumes variables dans chacune des chambres crées par ces dispositifs qui pourront varier de quelques microlitres, quelques millilitres, quelques centaines de millilitres voire quelques litres.

Sans que cela soit limitatif quant aux domaines, aux arrangements et aux tailles possibles de réalisations de ces dispositifs, certains exemples de ces tailles et de ces applications ressortiront à la lecture des exemples qui suivent.

Les applications thérapeutiques qui peuvent bénéficier du dispositif de notre invention sont nombreuses. Les efforts de la recherche aujourd'hui et les avancées notamment en oncologie offrent de plus en plus une médecine dites personnalisée ou chaque patient reçoit souvent un traitement pharmaceutique incluant plusieurs agents thérapeutiques injectables et à des doses et fréquence qui lui sont spécifiques.

Un des champs d'applications directe et immédiat de notre invention est certainement le domaine de l'insulinothérapie et les traitements du diabète en général dans lequel chaque injectable peut être choisi et adapté en fonction du patient et de son alimentation avec des possibilités de changement à chaque injection jusqu'à plusieurs fois par jours.

A titre d'exemples illustratifs mais non limitatifs sur les applications thérapeutiques ou prophylactiques des dispositifs connecteur-seringues selon l'invention, on peut citer le diabète, l'hormonothérapie, antibiothérapie, la cancérologie, immunologie, les vaccins, les traitements d'urgence. Cela s'applique à la médecine humaine mais aussi en vétérinaire où les injections sont souvent compliquées par exemple en antibiothérapie ou dans d'autres cas de traitements préventifs où l'on peut avoir recours à plusieurs produits injectables.

La présente invention a également pour objet l'utilisation d'un dispositif du type connecteur-seringue défini ci-dessus, caractérisée en ce que les produits à administrer sont utilisés dans les traitements du diabète, de l'infertilité, en hormonothérapie, en cancérologie, comme vaccins, ou dans tous les traitements dans lesquels plusieurs injections rapprochées doivent être pratiquées.

Les dispositifs selon l'invention permettent par exemple de combiner en une seule injection et à toutes les doses souhaitables l'insuline basale Glargine commercialisée dans les formulations Lantus®, Pasaglar® Toujeo® avec le peptide analogue du GLP-1 lixisenatide commercialisé dans la formulation Lyxumia® quelques soient leurs présentations ou dispositifs d'injection, en stylo-injecteurs ou en flacons.

Les dispositifs selon l'invention permettent l'injection de différents produits sans contact ou mélange avant injection entre ces produits. Cela apporte une solution aux problèmes de compatibilité et permet par exemple d'injecter en une seule piqûre des produits à pH physiologiques, ou proche de pH 7, avec un produit à pH acide susceptible de précipiter ou de se modifier à pH neutre, donc normalement incompatible avec ces autres produits et d'obtenir les réponses thérapeutiques de tous ces produits comme s'ils avaient été injectés séparément.

Ce qui importe souvent c'est que l'injectable touche les tissus corporels au site d'injection dans sa formulation définie pour obtenir son mécanisme de fonctionnement et cela ne dépend pas nécessairement ou uniquement du pH physiologique rencontré. Si le mélange avant injection est impossible par exemple parce qu'il entraine une évolution de chaque produit avant injection, le fait d'injecter les produits l'un après l'autre au même site sans mélanger avant leurs formulations ne présente pas les mêmes problèmes et peut être une réponse directe à ce problème. On peut aussi optimiser cela en respectant un temps d'attente entre chaque produit comme c'est déjà le cas avec beaucoup d'injectables. Il est également possible avec le dispositif selon l'invention et comme cela sera décrit dans les figures, de délivrer en une seule injection, un ou plusieurs produits à différentes profondeurs dans des zones tissulaires distinctes. Chacune de ces solutions peut aussi être une réponse à la douleur parfois provoquée par certains de ces produits tels que les injectables acides.

Chaque type d'insuline a des actions complémentaires et certains patients doivent s'injecter une insuline prandiale avant chaque prise alimentaire soit au moins trois injections par jour. Les dispositifs selon l'invention vont permettre de combiner par exemple l'injection d'abord de Lispro Humalog® puis celle de Glargine Lantus®, Basaglar ou Toujeo® ou encore de Detemir Deglutec®. On pourra ajouter aussi une insuline pré-mélangée ou premix comme NovoLog® ou encore par exemple, combiner Lantus® avec Victoza® ou tous autre GLP-1 ou peptides analogues.

Un avantage des dispositifs selon l'invention par exemple dans le cas des traitements du diabète est donc de réduire le nombre d'injections quotidiennes quel que soit le type d'insuline, ce qui donne plus de temps à chaque point d'injection pour récupérer de l'injection précédente avant de procéder à une nouvelle injection au même site. Même dans les cas où l'injection d'insuline basale doit être répétée deux fois par jour, ceci n'est plus un problème si ces deux injections disparaissent parce qu'on peut les combiner aux autres injections. Le fait de pouvoir combiner par exemple chaque traitement d'insuline basale avec d'autres injectables, GLP-1, insuline prandiale ou pré-mélangée est plus adapté à ces traitements multiples que les nouveaux produits sous forme de formulations ou de dispositifs en combinaisons fixes.

Ces nouveaux traitements combinés ouvrent la porte aux applications des dispositifs selon l'invention selon une même logique, notamment aux patients qui utilisent déjà ces produits à toutes les autres doses différentes des combos-formulations et qui sont soucieux de réduire leur nombre d'injection.

Les dispositifs selon l'invention s'appliquent aussi aux enfants et aux adolescents chez lesquels diminuer le nombre d'injection est clairement susceptible d'améliorer la compliance.

La présente invention a également plus particulièrement pour objet l'utilisation dans laquelle plusieurs injections sont pratiquées à l'aide d'un dispositif du type connecteur-seringue défini précédemment caractérisée en ce que au moins deux produits choisis dans au moins un des groupes a) à c) suivants sont administrés dans le traitement du diabète :
a) l'insuline ou d'un dérivé de l'insuline choisi de préférence parmi les insulines rapides, semi-lentes, lentes, basales ou mélanges d'insuline telles que les produit Tresiba®, Lantus®, Toujeo®, Humalog®, Umulin®, Levemir®, Basaglar®, Actrapid®, NovoMix®, Mixtard®, Apidra®, NovoRapid®,
b) le GLP-1 ou les peptides analogues du GLP-1 choisis de préférence parmi le Liraglutide (contenu par exemple dans le produit commercial Victoza®), la Lixisénatide (contenue par exemple dans le produit commercial Lyxumia®), l'Albiglutide (contenu par exemple dans le produit commercial Tanzeum®), l'Exenatide (contenue dans les produits commercial Byetta® ou Bydureon®), le Dulaglutide (contenu par exemple dans le produit commercial Trulicity®), le Semaglutide ou la Taspoglutide,
c) le Glucagon ou un Glucagon analogue, sous forme stable en solution ou sous forme solide ou lyophilisée à reconstituer.

Dans un mode d'exécution préféré de l'invention, la présente invention a également plus particulièrement pour objet l'utilisation d'un dispositif du type connecteur-seringue défini précédemment caractérisée en ce que au moins deux produits sont choisis dans au moins deux des groupes a) à c) précédemment indiqués.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en deux produits sont administrés et en ce que l'un des deux produits administré est l'insuline ou d'un dérivé de l'insuline choisi dans le groupe a) décrit ci-dessus et l'autre produit est le GLP-1 ou les peptides analogues du GLP-1 choisi dans le groupe b) décrit ci-dessus.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en ce que deux produits sont administrés et en ce que l'un des deux produits administré est l'insuline ou d'un dérivé de l'insuline choisi dans le groupe a) décrit ci-dessus et l'autre produit est l'insuline ou un dérivé de l'insuline choisi dans le même groupe a) décrit ci-dessus.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en deux produits sont administrés et en ce que l'un des deux produits administré est l'insuline ou d'un dérivé de l'insuline choisi dans le groupe a) décrit ci-dessus et l'autre produit est le Glucagon ou un Glucagon analogue choisi dans le groupe c) décrit ci-dessus.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en trois produits sont administrés et en ce que l'un des trois produits administré est l'insuline ou d'un dérivé de l'insuline choisi dans le groupe a) décrit ci-dessus, l'autre produit est le GLP-1 ou les peptides analogues du GLP-1 choisi dans le groupe b) décrit ci-dessus et le troisième produit est le Glucagon ou un Glucagon analogue choisi dans le groupe c) décrit ci-dessus.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en deux produits sont administrés et en ce que l'un des deux produits administré est le GLP-1 ou les peptides analogues du GLP-1 choisi dans le groupe b) décrit ci-dessus et le deuxième produit est le Glucagon ou un Glucagon analogue choisi dans le groupe c) décrit ci-dessus.

La présente invention a également plus particulièrement pour objet l'utilisation telle que décrite ci-dessus dans laquelle plusieurs injections rapprochées sont pratiquées à l'aide d'un dispositif du type connecteur-seringue défini ci-dessus, caractérisée en ce que lesdits produits à administrer sont utilisés dans les traitements du diabète et sont constitués d'une part de l'insuline ou d'un dérivé de l'insuline choisi de préférence parmi les insulines rapides, semi-lentes, lentes ou basales telles que les produit Tresiba® ou Lantus ® et d'autre part du GLP-1 ou les peptides analogues du GLP-1, tel que par exemple le liraglutide (contenu dans le produit commercial Victoza®), le Lixisenatide (contenu dans le produit commercial Lyxumia ®), l'Exenatide (contenu dans les produits commerciaux Byetta® ou Bydureon®) ou le Taspoglutide ; ou de glucagon ou d'un glucagon analogue, en combinaison avec un des peptides cités ci-dessus, plus particulièrement un analogue du GLP-1.

Ledit glucagon ou glucagon analogue peut aussi être utilisé dans les traitements d'urgence liés au diabète, telle que l'hypoglycémie, et du fait de son instabilité en solution, être conditionné dans le dispositif selon l'invention sous forme solide ou lyophilisée, et être reconstitué directement dans le réservoir juste avant administration.

La présente invention a également plus particulièrement pour objet l'utilisation dans laquelle plusieurs injections sont pratiquées à l'aide d'un dispositif du type connecteur-seringue défini précédemment caractérisée en ce que au moins deux produits choisis dans au moins un des groupes a) à c) suivants sont administrés dans le traitement de l'infertilité féminine :
a) Les GnRH (Gonadotropin-releasing hormone) agonistes, de préférence choisis entre les produits commerciaux Decapeptyl®, Suprefact® ou Synarel®
b) Les GnRH (Gonadotropin-releasing hormone) antagonistes, de préférence choisis entre les produits commerciaux Cetrotide® ou Orgalutran®
c) L'hormone folliculostimulante, de préférence choisis entre les produits commerciaux Gonal-F®, Puregon®, Menopur®, Pergoveris®, Fostimon®.

Dans un mode d'exécution préféré de l'invention, la présente invention a également plus particulièrement pour objet l'utilisation d'un dispositif du type connecteur-seringue défini précédemment caractérisée en ce que au moins deux produits sont choisis dans au moins deux des groupes d) à f) précédemment indiqués.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en deux produits sont administrés et en ce que l'un des deux produits administré est le GnRH agoniste choisi dans le groupe d) décrit ci-dessus et l'autre produit est l'hormone folliculostimulante choisi dans le groupe f) décrit ci-dessus.

Dans un mode d'exécution préféré de l'invention, la présente invention a également plus particulièrement pour objet l'utilisation dans laquelle plusieurs injections sont pratiquées à l'aide d'un dispositif du type connecteur-seringue défini précédemment, caractérisée en ce que deux produits sont administrés et en ce que l'un des deux produits administré est le GnRH antagoniste choisi dans le groupe e) décrit ci-dessus et l'autre produit est l'hormone folliculostimulante choisi dans le groupe f) décrit ci-dessus.

La présente invention a également plus particulièrement pour objet l'utilisation telle que décrite ci-dessus dans laquelle plusieurs injections rapprochées sont pratiquées à l'aide d'un dispositif du type connecteur-seringue défini ci-dessus, caractérisée en ce que lesdits produits à administrer sont utilisés dans les traitements de l'infertilité et sont constitués d'une part des GnRH agonistes choisi de préférence parmi les produits Decapeptyl®, Suprefact® ou Synarel®, ou les GnRH antagonistes choisi de préférence parmi les produits Cetrotide® ou Orgalutran®, et d'autre part de l'hormone folliculostimulante choisi de préférence parmi les produits Gonal-F®, Puregon®, Menopur®, Pergoveris®, Fostimon®.

La présente invention a également plus particulièrement pour objet l'utilisation telle que décrite ci-dessus d'un dispositif du type connecteur-seringue défini ci-dessus, caractérisée en ce que lesdits produits à administrer dans lesdits volumes sont sous forme solide, semi-solide, lyophilisée ou non et sont pré-chargés ou chargés au moment de l'utilisation dudit dispositif connecteur-seringue.

La présente invention a également pour objet un procédé de remplissage d'un dispositif connecteur-seringue défini ci-dessus, comprenant, à l'intérieur d'un corps externe réservoir (2) un élément interne piston creux (1), fermant ledit corps réservoir d'un côté, procédé caractérisé en ce que les produits à conditionner sont introduits à l'intérieur dudit réservoir (2) du côté d'une connexion distale (2a) ou du côté dudit piston creux (1) avant ou après que ledit piston creux (1) ne soit introduit dans ledit réservoir (2).

La présente invention a également plus précisément pour objet un procédé de remplissage d'un dispositif connecteur-seringue défini ci-dessus, comprenant, à l'intérieur d'un réservoir (2) cloisonné dans ses volumes par un piston plein (12) et par un piston creux (1), caractérisé en ce que les produits à conditionner sont introduits à l'intérieur dudit réservoir (2) du côté d'une connexion distale (2a) après que ledit piston plein ait été positionné dans ledit réservoir ou du côté dudit piston creux (1), avant ou après que ledit piston plein (12) ou ledit piston creux (1) ne soit introduit dans ledit réservoir (2).

La présente invention a ainsi pour objet un procédé de préparation et de conditionnement dudit ou desdits produits dans ledit connecteur-seringue tel que, sans limitation et à titre d'exemple sous forme de poudre ou de lyophilisé.

La présente invention a encore pour objet un procédé d'utilisation et de dispensation par lequel on remplit extemporanément ledit connecteur-seringue de la ou des quantités souhaitées de produits, on le connecte à un ou plusieurs dispositifs d'injection et on utilise ledit connecteur-seringue comme un dispositif d'injection complémentaire à travers une seule aiguille.

Selon l'invention, le dispositif connecteur-seringue est préférentiellement utilisé comme un réservoir que l'on peut remplir séparément des quantités ou volumes variables souhaités d'un ou plusieurs produits et qui, connecté à un ou à plusieurs dispositifs d'injection qui peuvent contenir des quantités ou volumes d'autres produits selon des ratios de volumes variables, isole les différents produits, évite leurs mélanges et permet leur injection via une seule aiguille.

La présente invention a également plus précisément pour objet un procédé par lequel on utilise ledit dispositif connecteur-seringue comme dispositif pour connecter des dispositifs d'injection pour injecter des quantités contrôlées du ou des produits à administrer et comme dispositif pour injecter le ou lesdits produits par une seule aiguille.

La présente invention a également pour objet d'améliorer le procédé d'implantation d'aiguille et d'écoulement du fluide. Le geste d'injection, lorsqu'effectué avec le dispositif de l'invention, permet aussi l'injection du produit contenu dans le réservoir distal selon un mécanisme amorti par un phénomène de « piston hydraulique », moins douloureux, ce qui permet d'améliorer la tolérance liée à l'injection.
Le phénomène de « piston hydraulique » vient de l'agencement du dispositif selon l'invention, composé d'un tube cylindrique, d'un piston et d'un orifice pour évacuer un fluide, qui vient faire pression sur ledit fluide et non sur un élément solide tel que dans un dispositif standard. Le fluide, de par sa viscosité inferieure, a une plus grande capacité d'amortissement, ce qui permet de réduire les forces d'injections vis-à-vis d'un élément solide, qui de par sa dureté possède un coefficient d'absorption moins élevé.

De plus, cet arrangement permet aussi de réguler la vitesse de pénétration de l'aiguille ou canule. Dans un montage classique de type seringue, la force est directement transmise à l'aiguille ; dans le cas d'une injection faite avec une force trop grande (par exemple un utilisateur inexpérimenté), ou trop brutale, cette force se répercute directement sur les tissus, entrainant de la douleur pour le patient. Concernant le montage selon le dispositif de l'invention, l'aiguille va entrer dans les tissus à une vitesse correspondant au processus de pénétration-coupure-lésions des tissus obtenus en fonction de la pointe et section du biseau : si la vitesse est trop élevée, l'effet « piston hydraulique » va amortir et ralentir cette pénétration. A contrario, si la vitesse de pénétration est trop lente ou n'est pas constante, le phénomène évitera fluctuation ou pénétration trop lente de l'aiguille.

Les dispositifs selon l'invention peuvent être obtenus par les techniques industrielles classiques connus de l'homme de l'art et par exemple par usinage ou thermo-moulage de pièces plastiques par exemple transparents ou de tout autre matériel compatible avec son utilisation.

Par ailleurs, comme indiqué ci-dessus, le dispositif selon l'invention peut être soumis à des processus de stérilisation pour assurer le maintien de l'asepsie au moment de son utilisation.

Les dispositifs connecteur-seringues selon l'invention peuvent être fabriqués en grande série à des coûts réduits selon des processus standards déjà connus et validés pour les nombreux connecteurs et dispositifs d'injections existants sur le marché. Il s'agit en général de fabrications par injection-moulage à partir des plastiques utilisés dans ces domaines.

On peut assembler les pièces constitutives des dispositifs selon cette invention de la même manière que celles par exemple des seringues jetables sur le même type de machines, en automatique et avec les mêmes précisions de positionnement-placement.

Comme dans ces exemples, on peut réaliser les pistons dans les caoutchoucs, les silicones ou les butyles habituellement utilisés. On peut réaliser des pistons séparés dans ces matières ou utiliser des joints portés par les pièces plastiques dans des gorges ou anneaux prévus à cet effet. On peut aussi utiliser des éléments de joints standards existants comme par exemple des joints o-ring. Comme dans ces exemples aussi de seringues ou comme dans le cas des aiguilles pour stylo-injecteurs, on peut associer à ces pièces plastiques des pièces métalliques telles que les aiguilles d'injection ou de percement des septums par exemple par surmoulage ou par collage dans ces pièces plastiques.

Ces pièces assemblées peuvent aussi être conditionnées selon des processus comparables, par exemple individuellement sous blister. Elles peuvent aussi être stérilisées avec les mêmes méthodes. On peut ainsi obtenir ces dispositifs en grandes quantités à des coûts compatibles avec leur utilisation unique et les réaliser dans les différentes tailles et avec les différents montages et connexions nécessaires selon le domaine de leurs applications.

Les développements qui suivent expliquent en détail et à titre non limitatif les caractéristiques de l'invention. Ces développements font référence à titre d'illustration aux figures ci-jointes. Ils ont cependant pour objet de décrire des modes d'exécution de l'invention qui ne se limitent pas auxdites figures.

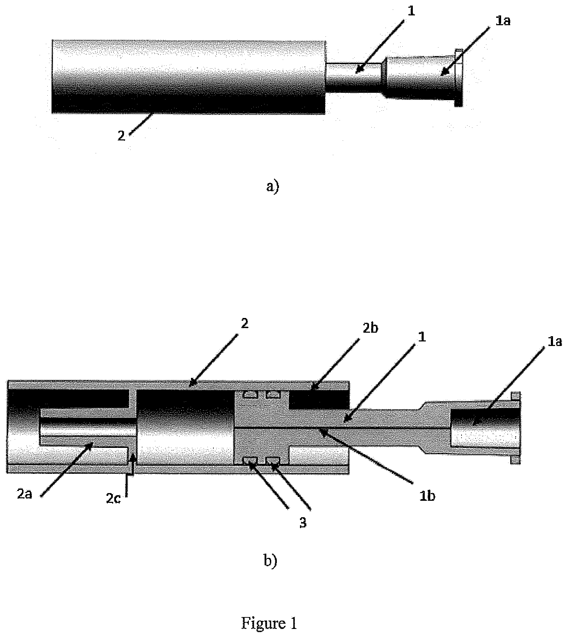

Par exemple, la figure 1 représente schématiquement un des modes d'exécution possibles du dispositif connecteur-seringue selon l'invention permettant l'administration en une seule injection de certaines combinaisons de produits.

Dans un mode d'exécution de l'invention, le piston creux (1) est terminé dans la partie amont extérieure au réservoir (2), par une connexion proximale (1a) de type femelle, luer ou luer-lock et le réservoir (2) est terminé dans la partie avale par une connexion distale (2a) de type mâle, luer ou luer-lock.

De manière générale, le piston creux (1) est troué ou ouvert en permanence d'un canal interne (1b) qui fait communiquer l'intérieur du réservoir (2) en aval par la partie interne de la connexion proximale (1a) vers l'extérieur du dispositif en amont.

Comme indiqué ci-dessus, le canal interne (1b) peut être de diamètre, de forme et de section variable mais sera préférentiellement réalisé rectiligne, du plus petit diamètre possible compatible avec le passage des produits utilisés dans le dispositif connecteur-seringue selon des forces et des pressions acceptables pour son utilisation par exemple manuelle, de façon à réduire les volumes morts résiduels dans ledit dispositif après son utilisation et à assurer au mieux la séparation des différents produits dans les phases de chargement ou d'administration.

Dans tous les cas et par exemple sur les autres versions représentées sur les autres figures, le piston creux (1) et le canal interne (1b) peuvent être décrits comme sur cette figure 1.

Dans la figure 1b, le piston creux (1) est représenté du côté aval à l'intérieur du réservoir (2) terminé par une tête de piston du type à deux joints (3) par exemple de type o-ring. Dans le cas de cette figure 1 et de manière plus générale, cette partie piston fonctionnel peut être réalisée selon toutes les approches classiques telles que par exemple avec un ou plusieurs joints indépendants sous forme d'anneaux cylindriques engagés dans une ou plusieurs gorges prévues à cet effet dans la tête de piston ou sous forme d'un piston butyle troué à une ou plusieurs lèvres positionnées autour de la tête de piston ou directement faire partie de la tête de piston par une surface lisse ou à un ou plusieurs anneaux de contact.

Par ces moyens ou par d'autres moyens adaptés, le piston creux (1) assure un contact et une étanchéité suffisante sur la paroi interne (2b) du réservoir (2) pour séparer et déplacer lesdits produits sans fuites. Par ces moyens aussi ou par d'autres moyens adaptés, on peut aussi contrôler les forces de fiction pour obtenir un ordre souhaité dans le mouvement des différentes pièces entre elles.

Par exemple, dans la figure 1b, le réservoir (2) est représenté ouvert en aval dans la connexion distale (2a) qui fait communiquer l'intérieur du réservoir (2) avec l'extérieur du dispositif en aval.

De manière générale, la connexion distale (2a) peut comporter à l'intérieur de sa partie externe dans le prolongement du corps externe du réservoir (2), un pas-de-vis spécifique non représenté ici, pour réaliser par rotation une connexion de type luer-lock avec un dispositif d'injection associé en aval. Cette partie externe de cette connexion distale (2a) peut aussi se prolonger comme représenté ici, de façon à assurer un point de contact de plus large surface avec la peau au moment de l'injection.

Le piston creux (1) est représenté ici introduit dans le réservoir (2) à une distance moyenne. Le piston creux (1) peut être déplacé à l'intérieur du réservoir (2) en conservant ladite étanchéité avec la paroi interne (2b), depuis son extrémité amont jusqu'à son extrémité avale au contact du fond (2c) du réservoir (2). Ces déplacements peuvent être opérés par des actions en translation, réalisées sur la connexion proximale (1a) du piston creux (1). Ces déplacements peuvent aussi être opérés sous l'influence des volumes desdits produits introduits ou extraits dudit réservoir.

De manière générale, on pourra par exemple positionner un bouchon luer femelle standard sur la connexion distale (2a) et une seringue standard remplie du volume d'un premier produit sur la connexion proximale (1a) et déplacer ledit piston creux depuis le fond (2c) jusqu'à une position plus amont dans ledit réservoir qui dépendra dudit volume transféré depuis ladite seringue standard. On pourra aussi par exemple positionner un bouchon mâle luer standard sur la connexion proximale (1a) et l'extrémité d'un cathéter connecté à un dispositif d'injection sur la connexion distale (2a) et déplacer ledit piston selon lesdits volumes à transférer.

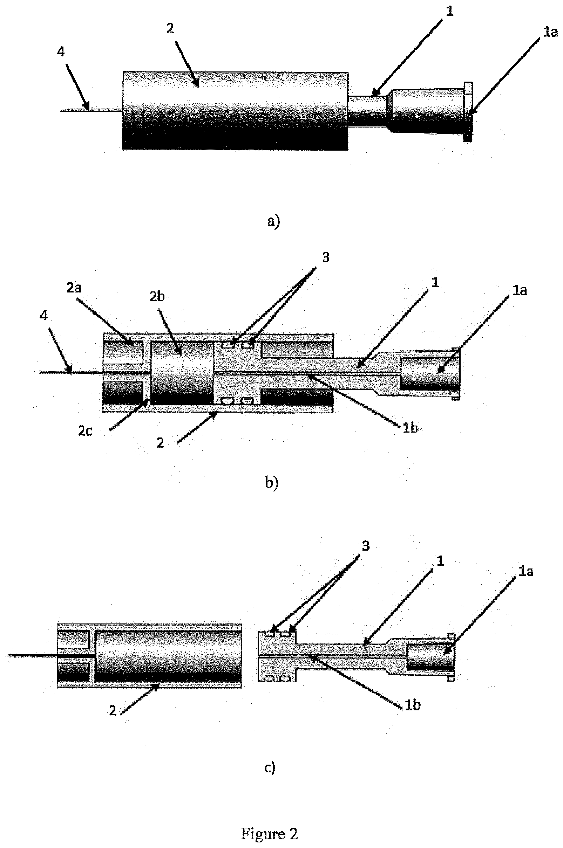

Par exemple, la figure 2 représente schématiquement un des modes d'exécution possibles du dispositif connecteur-seringue selon l'invention permettant l'administration en une seule injection de certaines combinaisons de produits.

Dans un mode d'exécution de l'invention, le piston creux (1) est terminé dans sa partie amont extérieure au réservoir (2), par une connexion proximale (1a) de type femelle, luer ou luer-lock et le réservoir (2) est terminé dans la partie avale par une connexion distale (2a) de type à aiguille fixe (4).

Le réservoir (2) est représenté ouvert en aval dans la connexion distale (2a) par une aiguille fixe (4) qui fait communiquer l'intérieur du réservoir (2) avec l'extérieur du dispositif en aval. Cette connexion distale (2a) peut comporter une partie externe qui peut se prolonger comme représenté ici, de façon à assurer un point de contact de plus large surface avec la peau au moment de l'injection.

Le piston creux (1) est représenté ici soit introduit dans le réservoir (2) à une distance moyenne (figure 2b) soit avant son introduction, à l'extérieur du réservoir (2) devant l'ouverture amont du réservoir (2) par laquelle il est introduit (figure 2c).

Cet assemblage a préférentiellement lieu au cours de la fabrication dudit dispositif, avant son emballage par exemple en blister individuel. Ledit piston creux (1) peut être déplacé à l'intérieur du réservoir (2) en conservant ladite étanchéité avec la paroi interne (2b), depuis son extrémité amont jusqu'à son extrémité avale au contact du fond (2c) du réservoir (2). Ces déplacements peuvent être opérés par des actions en translation, réalisées avec la connexion proximale (1a) du piston creux (1). Ces déplacements peuvent aussi être opérés sous l'influence des changements de volumes desdits produits à l'intérieur du réservoir (2).

On pourra par exemple positionner un bouchon butyle standard non représenté ici, sur l'aiguille fixe (4) de la connexion distale (2a) et une seringue standard remplie du volume d'un premier produit A sur la connexion proximale (1a) et déplacer ledit piston creux depuis le fond (2c) jusqu'à une position plus amont dans ledit réservoir qui dépendra dudit volume dudit produit A transféré depuis ladite seringue standard. On pourra aussi par exemple positionner un bouchon mâle luer standard sur la connexion proximale (1a), percer le bouchon septum d'un flacon et prélever un volume d'un premier produit A en actionnant le piston creux (1) par la partie amont de sa connexion proximale (1a). Dans les deux cas, on pourra ensuite fixer par exemple une seringue pré-remplie du volume d'un second produit B sur la connexion proximale (1a) et pratiquer l'injection en séquence des 2 produits A puis B, par la même aiguille fixe (4).

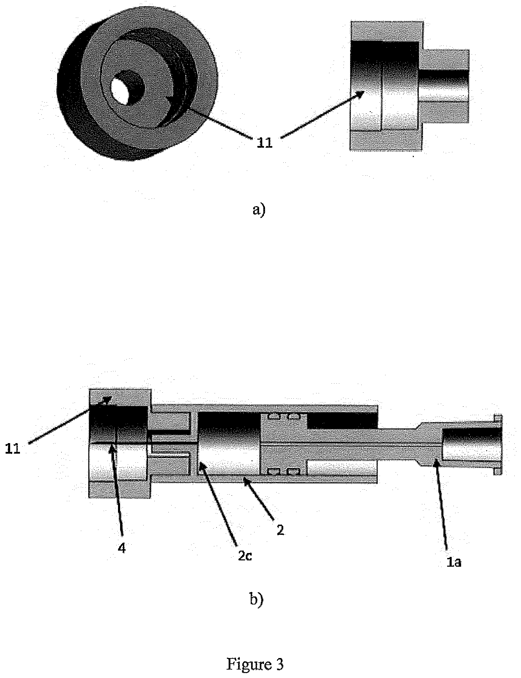

Par exemple, la figure 3b représente schématiquement le dispositif connecteur-seringue dans le mode d'exécution de la figure 2 associé à une pièce complémentaire ou connexion (figure 3a) séparée adaptateur stylo-injecteur (11) qui fait partie de l'invention.

Dans un mode d'exécution de l'invention, l'adaptateur stylo-injecteur (11) vient se positionner dans la connexion distale (2a) à l'extrémité avale du réservoir (2). Ainsi positionné, l'adaptateur stylo-injecteur (11) reproduit une connexion comparable aux connexions standards à vis et aiguille des stylo-injecteurs dans laquelle la partie aiguille est assurée par l'aiguille fixe (4).

Un mode général d'utilisation des dispositifs selon l'invention peut être décrit comme suit :
Si on a préalablement placé un bouchon luer mâle standard ou une seringue remplie d'un produit B (non représentés sur la figure 3), sur l'extrémité amont du piston creux (1) au niveau de sa connexion proximale (1a), on pourra alors visser n'importe quel stylo-injecteur sur cette extrémité avale du réservoir (2) pour charger une dose de produit A, contrôlée et transférée par ledit stylo-injecteur selon les mêmes étapes ou opérations de son protocole d'utilisation habituelle pour injecter directement cette dose de produit A. On pourra ensuite dévisser ledit stylo-injecteur, retirer ledit adaptateur stylo-injecteur et pratiquer l'injection avec ladite seringue remplie d'un produit B fixée sur ladite connexion proximale (1a) en implantant l'aiguille fixe (4) au point d'injection choisi. On injectera alors par exemple d'abord le produit A, en tenant ladite seringue au niveau de son appui-doigt et de la base de sa tige de piston et en enfonçant le piston creux (1) dans le réservoir (2) jusqu'à ce qu'il touche le fond (2c) et collapse le réservoir (2). On injectera ensuite le produit B, à partir de ladite seringue remplie, selon le protocole de son utilisation habituelle, par action entre ledit appui-doigt et ladite tige de piston. On aura ainsi injecté le produit A puis le produit B, en un seul point d'implantation de l'aiguille fixe (4) sans contact et sans mélanger les deux produits. Si nécessaire, on pourra respecter un temps précis entre l'injection du produit A et celle du produit B en marquant une pose contrôlée entre ces étapes, comme cela se pratique déjà pour de nombreuses injections.

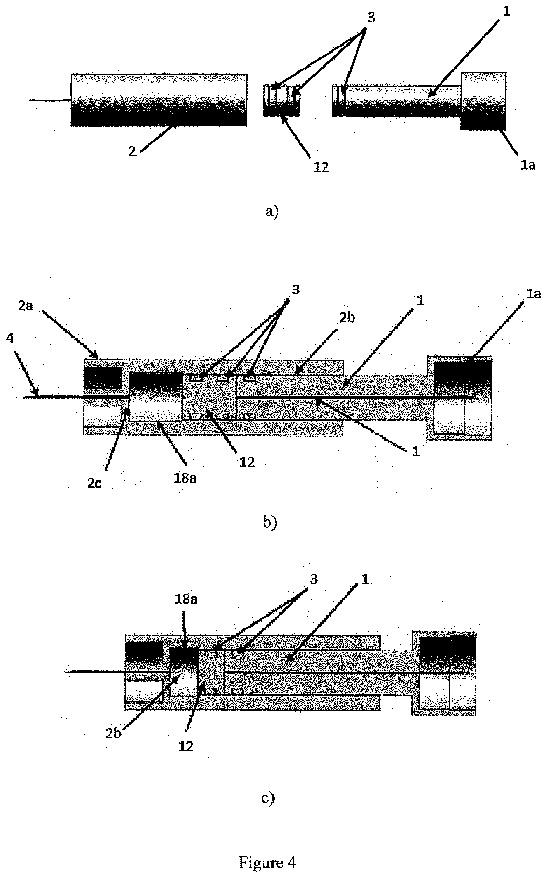

Par exemple, la figure 4 représente schématiquement le dispositif connecteur-seringue dans un mode d'exécution comportant un piston plein (12) et un piston creux (1) selon un des arrangements possibles dudit dispositif connecteur-seringue selon l'invention pour permettre l'administration de deux, trois ou plus de trois produits en une seule injection sans mélanger et sans contact entre produits selon certaines combinaisons possibles de ces produits.

Dans un mode d'exécution de l'invention, le piston creux (1) est terminé dans sa partie amont extérieure au réservoir (2), par une connexion proximale (1a) du type standard à vis et aiguille comme celle des aiguilles pour stylo-injecteurs et le réservoir (2) est terminé dans la partie avale par une connexion distale (2a) de type à aiguille fixe (4).

Le piston creux (1) est troué ou ouvert en permanence d'un canal interne (1b) qui fait communiquer en aval, une partie de l'intérieur du réservoir (2) comprise entre ce piston creux (1) et le piston plein (12) avec en amont, la partie interne de la connexion proximale (1a) vers l'extérieur dudit dispositif. Ce canal interne (1b) peut par exemple être décrit et réalisé de la même manière et avec les mêmes objectifs que sur les figures 1 et 2.

Le piston creux (1) est représenté du côté aval à l'intérieur du réservoir (2) terminé par une tête de piston du type à un joints (3) par exemple type o-ring. Cette partie tête de piston peut par exemple être décrite et réalisée de la même manière et avec les mêmes objectifs que sur la figure 1.

Le réservoir (2) est représenté ouvert en aval dans la connexion distale (2a) par une aiguille fixe (4) qui fait communiquer l'intérieur du réservoir (2) avec l'extérieur du dispositif en aval. Cette connexion distale (2a) peut comporter une partie externe qui peut se prolonger comme représenté ici, de façon à assurer un point de contact de plus large surface avec la peau au moment de l'injection.

Le piston plein (12) est représenté sur la figure 4b et la figure 4c introduit dans le réservoir (2) en aval du piston creux (1) à la position précise qu'il doit occuper après assemblage ou montage des différentes parties dudit dispositif au moment de la fabrication et avant utilisation. Le piston creux (1) est représenté dans une position préférentielle avant utilisation, au contact à l'arrière du piston plein (12). Le piston plein (12) est dans une position qui peut lui permettre de remonter vers l'amont à l'intérieur du réservoir (2) si nécessaire, selon le ou les volumes de produits chargés dans le réservoir (2) par sa connexion distale (2a) au moment du remplissage dudit dispositif et selon un déplacement qui peut entrainer le piston creux (1) qui le touche en amont. Dans cette configuration aucun contact n'est possible entre les produits et leurs volumes chargés en aval du piston plein (12) et ceux chargés en amont de ce piston (12). Dans le réservoir (2) en aval de ladite position précise du piston plein (12) on trouve une partie dans laquelle la paroi interne (2b) du réservoir (2) présente une variation de diamètre de la paroi interne (18a) qui fait que lorsque le piston plein (12) se déplace vers l'aval de ladite position précise, les volumes et produits en aval et en amont du piston plein (12) peuvent être mis en communication d'une façon qui permette par exemple leurs transferts autour du piston plein (12) pour leur administration à travers l'aiguille fixe (4).

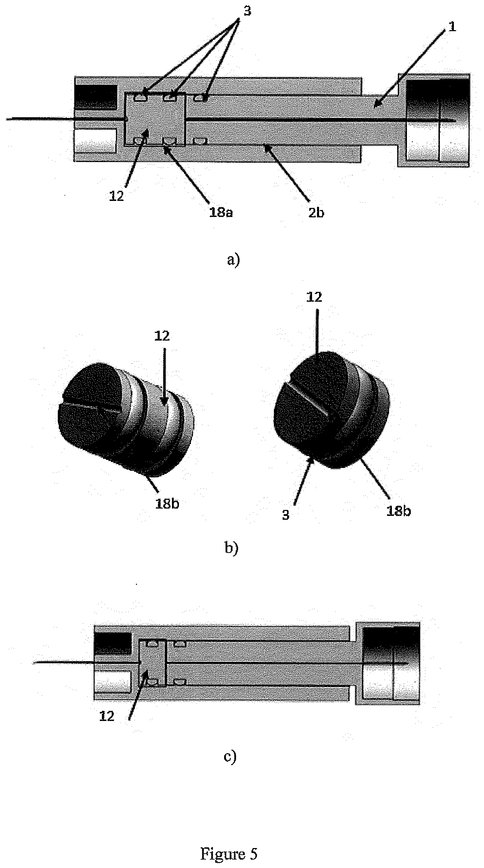

Sur les figures 4b et 4c, le piston plein (12) est représenté à sa position précise à l'intérieur du réservoir (2) dans deux configurations de piston avec joints séparés par exemples de type o-ring, d'abord avec deux joints (3) puis avec un seul joint (3) de façon à limiter les volumes morts de produits après utilisation dudit dispositif comme cela est précisé ensuite avec la figure 5.

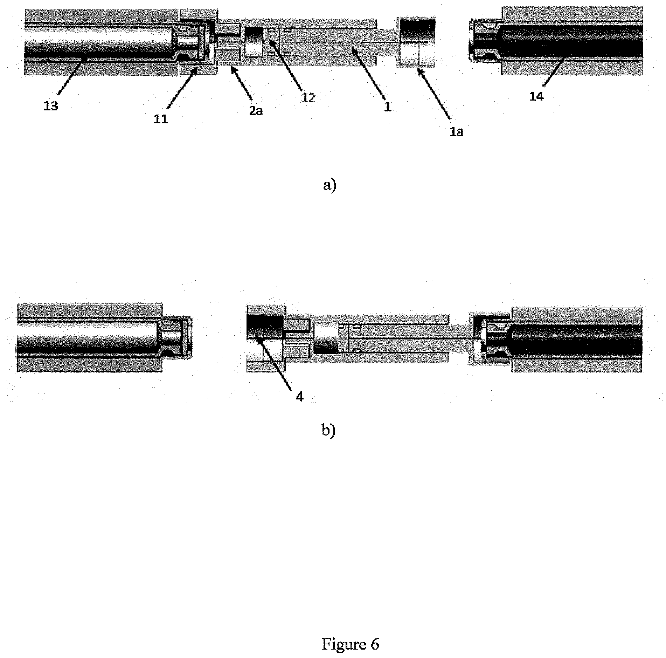

Ce piston plein (12) peut être réalisé selon toutes les approches classiques telles que par exemple comme ici, avec un ou plusieurs joints indépendants sous forme d'anneaux cylindriques engagés dans une ou plusieurs gorges prévues à cet effet dans la tête de piston ou sous forme d'un piston plein butyle à une ou plusieurs lèvres autour de la tête de piston tel que par exemple les piston intermédiaire des seringues bi-compartimentales avec by-pass ou encore comme une pièce plastique moulée avec une surface lisse ou à un ou plusieurs anneaux de contact. Par ces moyens ou par d'autres moyens adaptés, le piston plein (12) assure un contact et une étanchéité suffisante sur la paroi interne (2b) du réservoir (2) pour séparer et déplacer les volumes desdits produits. Un exemple de l'utilisation de ce type de dispositif connecteur-seringue avec piston plein (12) à partir de deux stylo-injecteurs pour injecter 2 produits en une seule piqûre sera précisé ensuite avec la figure 6.

Un autre mode général d'utilisation des dispositifs selon l'invention peut être décrit comme suit :
Si on souhaite par exemple injecter en une seule piqûre les doses de trois produits différents A, B et C dont par exemple un, A, est totalement incompatible avec les deux autres B et C, on peut utiliser ce type de dispositif connecteur-seringue avec piston plein (12) dans une version dans laquelle la connexion proximale (1a) du piston creux (1) est de type luer ou luer-lock, de façon à pouvoir y fixer des seringues standards. Dans ce mode d'exécution, si on ajoute sur la connexion distale (2a) un adaptateur stylo-injecteur (11), on peut charger d'abord un volume de produit A à partir d'un stylo-injecteur standard vissé sur l'adaptateur stylo-injecteur (11) dans cette connexion distale (2a) du côté aval, on peut ensuite connecter une première seringue qui contient le volume de produit B et le charger entre le piston plein (12) et le piston creux (1). On déconnecte ensuite cette première seringue pour connecter une seconde seringue chargée du produit C. On retire l'adaptateur stylo-injecteur (11) en aval et on injecte à travers l'aiguille fixe (4) d'abord le produit A en actionnant le piston creux (1) par son extrémité proximale (1a). On peut ensuite marquer une pause suffisante pour que ce produit A soit efficace ou diffuse depuis son point d'injection puis injecter le produit B en actionnant le piston creux (1) à partir de la seringue maintenue au niveau de son appui-doigt et de sa tige de piston. On peut si nécessaire marquer une seconde pause. On injecte ensuite le produit C en actionnant la seringue de façon standard.

Dans un autre mode d'exécution du dispositif connecteur-seringue avec piston plein dans laquelle la connexion distale (2a) du réservoir (2) est aussi de type luer ou luer-lock, on pourra réaliser la même opération et injecter trois produits contenus par exemple dans trois flacons à partir de trois seringues. La première seringue connectée en aval sur la connexion distale (2a) sera alors déchargée dans ledit dispositif par exemple à travers un autre connecteur standard femelle-femelle de type luer ou luer-lock. On pourra par exemple ne déconnecter cette première seringue en aval qu'après avoir rempli ledit dispositif du contenu de la seconde seringue et avoir connecté en amont la troisième seringue avec son contenu. On pourra alors par exemple fixer une aiguille standard ou un cathéter standard terminé par une aiguille sur la connexion distale (2a) dudit dispositif et pratiquer l'injection des trois produits comme précédemment, en un seul point sans les mélanger. Ces procédés seront les mêmes pour l'injection de plus de trois produits avec la possibilité d'avoir aussi plus de pistons intermédiaires permettant la séparation des différents produits liquides semi-solides ou solides.

Par exemple, la figure 5a et la figure 5c représentent schématiquement des détails d'un arrangement possible du réservoir (2) et du piston plein (12) dans sa position dite de by-pass au cours de l'utilisation ou après utilisation du dispositif connecteur-seringue selon l'invention.

Dans un mode d'exécution de l'invention, lorsque le piston plein (12) a fini son déplacement étanche à l'intérieur du réservoir (2) de l'amont vers l'aval du réservoir (2), il peut arriver au contact du fond (2c) dans une partie du réservoir (2) où il finit sa course et où il perd son étanchéité avec la paroi interne (2b) du réservoir (2) grâce à une variation de la section de la paroi interne (18a). Cette variation de section de la paroi interne (18a) est suffisamment grande pour permettre au volume du ou des produits en amont du piston plein (12) de s'écouler vers l'aval dudit dispositif avec des forces et pressions utilisables manuellement. Cette variation de la section interne (18a) est suffisamment petite pour ne pas constituer un volume mort perdu trop important pour ce ou ces produits. Cette variation de la section de la paroi interne (18a) pourra par exemple offrir une ouverture de section comparable à celle du canal interne (1b) ou des aiguilles de transfert ou d'injection utilisable dans chaque configuration dudit dispositif pour chaque type d'application.

Comme indiqué précédemment, cette variation de la section de la paroi interne (18a) a pour but de rompre l'étanchéité entre le piston plein (12) et la paroi interne (2b) du réservoir (2). Comme également indiqué précédemment elle pourra par exemple être obtenue par une légère augmentation du diamètre interne du réservoir (2), Cette rupture de l'étanchéité pourra aussi être obtenue par une déformation du cercle de la section de ce réservoir (2).

La présente invention inclut également toutes autres variations capables de rompre l'étanchéité entre le piston plein (12) et la paroi interne (2b) du réservoir (2) telle qu'au moins une gorge ou une rainure le long de la paroi interne (2b) sur une longueur donnée à partir du fond (2c) du réservoir (2), par exemple.

Par exemple, la figure 5b représente aussi schématiquement un exemple de variation de surface du piston plein (18b). Lorsque le piston plein (12) arrive au fond (2c) du réservoir (2), il peut être nécessaire d'avoir une variation de surface du piston plein (18b) qui va éviter que le piston plein (12) ne vienne boucher la sortie avale du réservoir (2) à travers la connexion distale (2a) et par exemple dans le cas représenté ici d'une sortie par aiguille fixe (4).

Dans un mode d'exécution de l'invention, pour permettre le passage du volume du ou des produits situés en amont du piston plein (12) on peut réaliser une variation de surface du piston plein (18b) sous forme d'au moins une gorge ou rainure.

On pourra par exemple assurer que la profondeur de celle-ci soit suffisante pour que l'ensemble du flux de volume du ou des produits situés en amont du piston plein (12) puisse s'écouler sans que ce passage n'entraine une force ou une pression supérieure à celle rencontrée dans les autres parties dudit dispositif. Cette variation de surface du piston plein sera aussi avantageusement la plus petite ou la plus fine possible pour limiter les volumes morts.

Par exemple, la figure 5b représente deux versions schématiques de cette variation de surface du piston plein (18b) avec un piston plein (12) à deux joints (3) et un second à un joint (3) susceptible de limiter lesdits volumes morts.

Par exemple, la figure 4a représente aussi schématiquement une vue éclatée des différentes pièces dudit dispositif avant montage. Le piston plein (12) est représenté entre le réservoir (2) et le piston creux (1), à l'extérieur du réservoir (2) devant l'ouverture amont du réservoir (2) par laquelle il est introduit. Selon un ordre d'assemblage préférentiel, on introduira en fabrication, d'abord le piston plein (12) dans le réservoir à la position précise par exemple avant la zone de by-pass, qu'il occupe avant utilisation dudit dispositif puis le piston creux préférentiellement au contact du piston plein (12). Cette dite vue éclatée permet aussi de mieux visualiser une possible variation de surface de la paroi interne (18a) de ladite zone de by-pass sur la paroi interne (2b) du réservoir (2).

Selon un mode d'exécution de ce type, le diamètre interne du réservoir (2) sous forme cylindrique augmente suffisamment pour que le piston plein (12) ne soit plus étanche avec la paroi (2b) et que les volumes du ou des produits en amont du piston plein (12) puissent s'écouler vers l'aval selon des forces et des pressions par exemple compatibles avec leur injection manuelle.

Par exemple, la figure 6 représente schématiquement un des dispositifs connecteur-seringues de la figure 4 dans une version avec un piston plein (12) et un piston creux (1) dans le réservoir (2). Le piston creux (1) est terminé dans sa partie amont par une connexion proximale (1a) du type standard à vis et aiguille comme celle des aiguilles pour stylo-injecteurs et le réservoir (2) est terminé dans la partie avale par une connexion distale (2a) de type à aiguille fixe (4) dans laquelle est positionnée un adaptateur stylo-injecteur (11).

Dans un mode d'exécution de l'invention représenté sur la figure 6a, un stylo-injecteur A (13), contenant un produit A par exemple incompatible avec un produit B est vissé sur cet adaptateur stylo-injecteur (11) et un volume du produit A préalablement sélectionné et contrôlé par ledit stylo-injecteur A (13) est chargé par actionnement dudit stylo-injecteur A (13) dans le réservoir (2) en aval du piston plein (12). Ledit volume selon son importance, peut déplacer le piston plein vers l'amont dans le réservoir (2). Ledit stylo A (13) est ensuite dévissé de l'adaptateur stylo-injecteur (11) et un deuxième stylo-injecteur B (14) contenant un produit B, tel que représenté sur la figure 6b, avec un volume préalablement sélectionné et contrôlé par ce stylo-injecteur B (14) est vissé sur la connexion proximale (1a). On peut alors retirer l'adaptateur stylo-connecteur (11) et pratiquer l'injection du produit A puis du produit B selon des opérations comparables à celles décrites précédemment et proches des standards d'utilisation de ces dispositifs d'injection. Pendant la préparation avant l'injection, l'adaptateur stylo-injecteur (11) a aussi joué le rôle de protecteur d'aiguille. Dans cet exemple on peut piquer l'aiguille (4) dudit dispositif connecteur-seringue comme on le ferait pour une aiguille de stylo-injecteur puis pousser sur le corps dudit stylo-injecteur B (14) pour collapser ledit dispositif et injecter ledit produit A. On pourra ensuite marquer une pause nécessaire à l'efficacité dudit produit A puis injecter ledit produit B comme on le fait habituellement avec ledit stylo-injecteur B (14). On retire ensuite l'ensemble dispositif connecteur-seringue et stylo-injecteur B (14) du point d'injection. On dévisse ledit dispositif dudit stylo-injecteur B (14) par sa connexion proximale (1a) et on élimine ledit dispositif comme une aiguille standard de stylo-injecteur par exemple en la recapuchonant avec l'adaptateur stylo-injecteur (11), avant de la dévisser. On peut ensuite stocker lesdits stylo-injecteurs A (13) et B (14) comme normalement recommandé avant leur prochaine utilisation sans risque de contamination croisée par lesdits produits A et B. Lesdits volumes A et B pourront être adaptés à chaque cas ou chaque traitement comme lors de leur utilisation habituelle séparée et injectés en une seule fois sans qu'il n'y ait jamais de contact ni donc de risque entre ces produits.

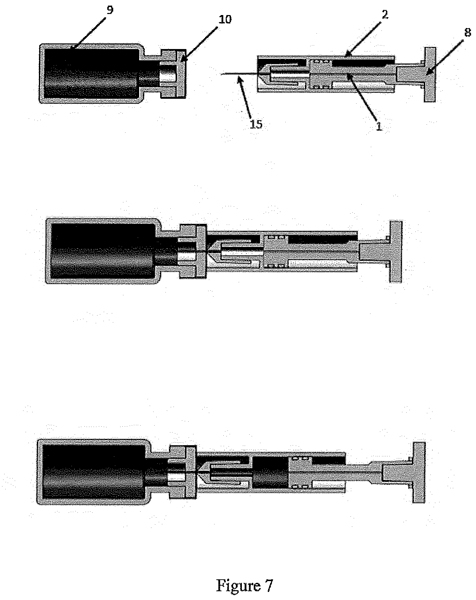

Par exemple, la figure 7 représente schématiquement une situation dans laquelle un dispositif connecteur-seringue selon l'invention est connecté à une aiguille standard (15) sur l'extrémité luer ou luer-lock de la connexion distale (2a).

Dans un mode d'exécution de l'invention, si un bouchon mâle luer standard (8) est fixé sur la connexion proximale (1a), on peut utiliser ledit dispositif pour prélever le volume d'un premier produit A, par exemple à travers le bouchon-septum (10) à l'intérieur d'un flacon (9) multi-doses de produit A. Le réservoir (2) est alors chargé du volume de ce premier produit A à injecter. On déconnecte alors ledit dispositif du flacon (9), on peut garder ou changer l'aiguille standard (15). Si on retire ensuite ledit bouchon mâle luer standard (8) et si une seringue luer ou luer-lock, chargée du volume d'un second produit B à injecter est connectée à l'extrémité luer de la connexion proximale (1a), on peut implanter l'aiguille standard (15) et pratiquer d'abord l'injection du premier produit A puis celle du second produit B, sans mélanger les produits A et B, en un seul point d'insertion sur la peau de ladite aiguille standard (15). L'air contenu dans le canal interne (1b) va permettre de garder les produits A et B séparés jusqu'à leur injection.

On opère dans ce cas avec ledit dispositif comme on le ferait avec une seringue standard en utilisant le piston creux (1) par son extrémité proximale (1a) comme si c'était une tige de piston et par exemple ici, en piquant l'aiguille (15) à travers le bouchon-septum (10) d'un flacon (9) pour prélever le volume souhaité du produit A. Préférentiellement dans ce cas le corps du réservoir (2) est transparent et gradué de façon à doser ledit volume dudit produit A transféré dans ledit dispositif par la translation du piston creux (1). Une autre solution peut être de graduer le piston creux (1) sur sa surface extérieure d'une échelle qui permette de contrôler ledit volume prélevé même dans un réservoir non transparent. Une échelle graduée sur l'extérieur du piston creux (1) peut aussi être dans toutes les applications dudit dispositif, un moyen de vérifier le ou les transferts de volumes et donc de contrôler le fonctionnement dudit dispositif et du ou des dispositifs d'injection qui lui sont associé. Dans le cas représenté ici, une fois ledit dispositif chargé d'un volume du produit A prélevé dans le flacon (9) et l'aiguille standard (15) retirée du bouchon-septum, on pourra éventuellement aussi utiliser ledit dispositif comme une seringue et injecter directement son contenu en produit A à travers l'aiguille standard (15).

Si comme décrit précédemment, on retire ledit bouchon mâle luer standard (8) pour fixer à sa place par exemple une seringue chargé d'un autre produit B, on pourra injecter les deux produits A et B l'un après l'autre selon les étapes également décrites.

Dans ce cas comme dans tous les cas dans lesquels un premier produit A est prélevé du coté aval, ce produit A ne remplit pas le canal interne (1b) du piston creux qui reste en tout ou en partie rempli d'air. Si on connecte ensuite sur la connexion proximale (1a) du piston creux (1) un autre dispositif d'injection chargé d'un produit B, il n'est pas nécessaire de purger les quantités résiduelles d'air et l'injection peut se faire directement. Dans ce cas les produits A et B peuvent ne pas être mis en contact et rester séparés de la couche d'air du canal interne (1b). Même si on connecte en amont sur la connexion proximale un dispositif d'injection multi-doses du produit B son contenu ne sera pas contaminé par le produit A et ledit dispositif d'injection pourra être réutilisé plusieurs fois comme il l'est habituellement.

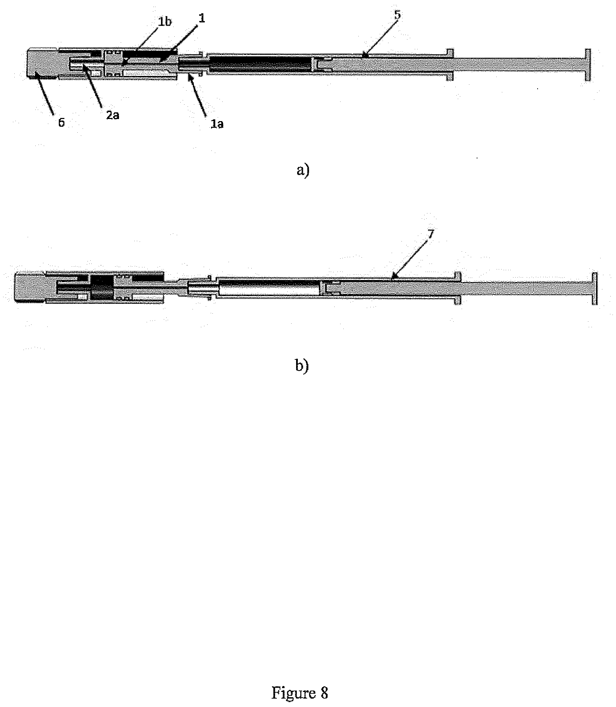

Par exemple, la figure 8 représente schématiquement une utilisation d'une version du dispositif connecteur-seringue dans laquelle ledit dispositif est du type représenté sur la figure 1 avec un bouchon femelle luer standard (6) en aval du réservoir (2) et une connexion proximale (1a) luer en amont sur laquelle est d'abord fixée une seringue de produit A. Dans cet exemple le bouchon femelle luer standard (6) est positionné de façon à obturer la connexion distale (2a) comme cela se fait sur les seringues pré-remplies sans aiguille.

Dans un mode d'exécution de l'invention, une fois connecté sur la connexion proximale (1a) l'embout luer d'une seringue remplie d'un produit A, on peut actionner normalement cette seringue et charger une dose de produit A contrôlée par celle-ci, tel que représenté sur la figure 8a. Le produit A passe par le canal interne (1b) et remplit le réservoir (2) dudit dispositif en déplaçant le piston creux (1) vers l'amont. On déconnecte ensuite la seringue de produit A et on connecte à la même extrémité amont une seringue de produit B (7), tel que représenté sur la figure 8b, on peut alors retirer le bouchon femelle luer standard (6) puis fixer sur cette connexion distale (2a), une aiguille standard et pratiquer l'injection de A et B en une seule fois selon des étapes décrites pour les figures précédentes. Cette aiguille standard n'aura pas préalablement percé de septum avant de pénétrer dans la peau. Dans ce cas on peut avoir un certain contact entre les produits A et B mais uniquement à l'intérieur dudit dispositif et sans risque de contact dans d'autres réservoirs qui pourra contenir ces deux produits si on utilise deux seringues différentes.

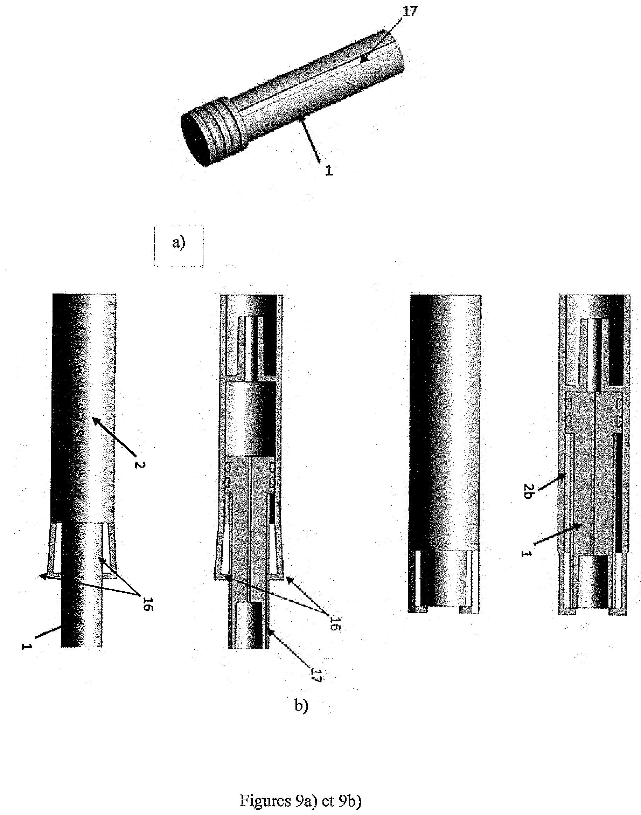

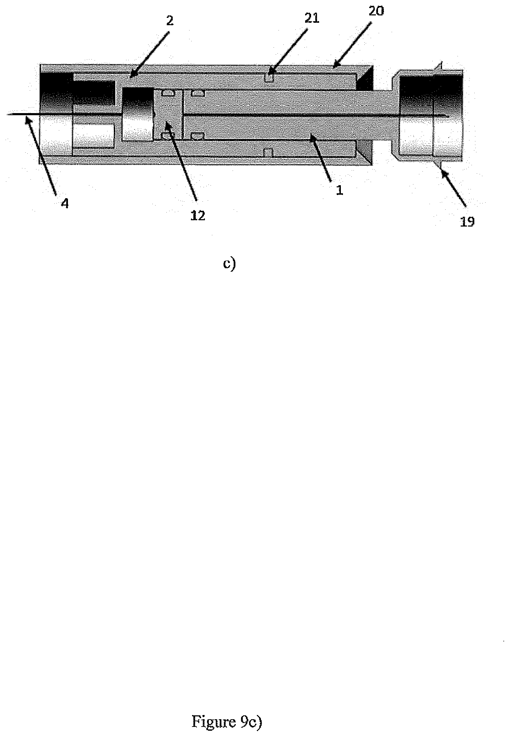

Par exemple, la figure 9 représente deux versions schématiques du dispositif connecteur-seringue équipé d'un système de blocage ou de verrouillage qui s'active en fin d'utilisation ou pendant l'injection, qui peut éviter ainsi que ledit dispositif puisse être réutilisé et qui peut contrôler la profondeur d'injection. Ledit système peut être obtenu selon différents arrangements ou adaptations possibles sur le seul corps externe réservoir (2), il peut aussi être obtenu avec des arrangements ou adaptations à la fois sur le réservoir (2) et sur le piston creux (1), il peut aussi être obtenu à l'aide d'une pièce supplémentaire sur le réservoir (2).

Dans un autre mode d'exécution de l'invention, un système de clapets de sécurité (16) porté par le réservoir (2) et de rigole (17) à la surface externe du piston creux (1) dans lesquels ces clapets de sécurités (16) se déplacent. Au début de l'utilisation, le piston creux (1) pourra être dans une position enfoncée dans le réservoir (2) mais sans contact avec le fond (2c), position dans laquelle il n'arrivera qu'à la fin de l'utilisation. De cette manière le piston creux (1) peut être librement déplacé vers l'amont au cours de la ou des premières phases d'utilisation dudit dispositif. Il peut aussi être librement déplacé vers l'aval au cours de la ou des phases suivantes d'utilisation. Dans ces différentes phases les clapets de sécurité (16) circulent librement dans les deux sens dans les rigoles (17). À la fin de l'utilisation dudit dispositif, le piston creux (1) atteint la position plus profonde dans le réservoir (2) au contact du fond (2c), dans laquelle les clapets de sécurité (16) sortent des rigoles (17) pour se refermer au-dessus du piston creux (1) et bloquer tous ses déplacements dans le réservoir (2) comme représenté sur cette figure 9b.

Dans un autre mode d'exécution de l'invention représenté sur la figure 9c, le dispositif comporte une connexion proximale (1a) équivalente à la tête d'une aiguille de stylo-injecteur et donc capable de se fixer sur tous les stylo-injecteurs. Dans ce mode d'exécution, une pièce supplémentaire, contrôle de profondeur (19) est ajoutée sur le réservoir (2). Pendant toutes les étapes d'utilisation dudit dispositif jusqu'à l'injection, cette pièce contrôle de profondeur (19) est verrouillée sur la partie amont du réservoir (2). Ce contrôle de profondeur (19) ne permet alors d'injecter qu'une partie de la longueur de l'aiguille fixe (4). Après injection du ou des produits avals, lorsque l'injection va collapser ledit réservoir (2) par déplacement du piston plein (12) au contact du fond (2c), ladite connexion proximale (1a) va libérer ledit contrôle de profondeur (19) de sa position verrouillée à la surface dudit réservoir (2) en sortant de leur habitacle les encoches de retenue (20) et ladite aiguille fixe (4) va pouvoir pénétrer plus profondément les tissus au site d'injection pour administrer le ou les produits amont. De cette façon, sans aucune manipulation dudit dispositif, on peut séparer les sites d'injection de différents produits administrés avec ledit dispositif selon l'invention. Ledit contrôle de profondeur (19) peut retrouver une seconde position de blocage grâce à des crochets de sécurité (21) à la surface de ladite connexion proximale qui va aussi servir à verrouiller ledit dispositif après son utilisation et empêcher ainsi toute réutilisation.

Les exemples suivants vont illustrer des possibilités d'utilisation du dispositif connecteur-seringue selon certains modes opératoires possibles. Le dispositif selon l'invention peut notamment être décliné en différentes versions et dimensions en fonction des caractéristiques des produits à injecter.

La première (illustrée mais non limitée par exemple par la Figure 2) est constituée d'un seul piston creux (1) percé d'un canal interne (1b) terminé en connexion proximale (1a) par exemple par un embout femelle luer qui se déplace par rapport à la paroi interne (2b) du réservoir (2) pour créer une chambre entre ce piston creux (1) et le fond (2c) du réservoir (2) comportant une aiguille (4) permettant l'injection de médicaments.

Cette première version peut être utilisée par exemple pour des connexions seringue-flacon, seringue-seringue et seringue-stylo-injecteur. Si nécessaire, une barrière contre une possible contamination entre un produit B contenu dans le dispositif d'injection connecté et le produit A contenu dans la chambre du dispositif connecteur-seringue peut être assurée par l'air notamment dans le canal interne (1b) qui sépare les deux produits.

La seconde version (illustrée mais non limitée par exemple par la Figure 4) possède un piston plein (12) en aval du piston creux (1) qui représente une barrière physique contre l'éventuelle contamination du dispositif contenant le fluide B par le fluide A contenu dans le réservoir (2), et qui permet dans certaines dispositions l'administration d'un troisième fluide C à travers la même aiguille (4).

### PARTIE EXPERIMENTALE

### Exemple 1 : Mise en évidence du fonctionnement et de la précision des administrations réalisées avec un dispositif selon l'invention dans une première configuration

### Préparation d'une solution de Peptide 1 à 200 µg/mL :

Approximativement 50mg de Peptide 1 sont pesés puis solubilisés dans 5mL d'acide acétique à 0,1% dont le poids est aussi déterminé par pesée. Par une dilution au 1/10 à partir de cette première solution à 2mg/mL est obtenue la solution finale à 200µg/mL.

Le peptide 1 est facilement détectable par analyse HPLC à faible concentration, stable une fois solubilisé dans une solution d'acide acétique à 0,1% et ses propriétés sont connues de l'homme du métier.

### Solution d'insuline humaine lente (Umuline® NPH) à 100UI/mL :

Cette solution est une solution commerciale d'insuline humaine commercialisée par la société ELI LILLY sous le nom de Umuline® NPH. Ce produit est une insuline à action intermédiaire. La solution est contenue dans un flacon commercial de 10mL à une concentration de 100UI/mL.

### Préparation du dispositif selon l'invention pour une solution d'insuline à 100UI/mL et une solution de peptide à 200µg/mL à partir d'un flacon et d'une seringue :

Dans cette configuration, le dispositif est équipé d'un bouchon luer mâle standard (non représenté sur la figure 2, visible en (8) sur la Figure 7), positionné au bout de la connexion proximale (1a) du piston creux (1) permettant de transformer le piston creux en un piston fermé. Cette pièce mâle conique et standard, se fixe dans cet embout femelle luer-lock. De cette manière, il est possible d'utiliser le dispositif comme une seringue pour prélever un volume équivalent à 5UI (50µL) de Umuline® NPH directement du flacon commercial de 10mL.

La paroi du réservoir (2b) étant transparente, un premier contrôle visuel est effectué pour vérifier que la dose d'insuline prélevée se retrouve entièrement dans la chambre distale du réservoir et que l'aiguille (4) du dispositif n'est pas obturée. La paroi du réservoir (2) ou le piston creux (1) est équipé d'une graduation permettant de connaitre le volume de produit introduit dans le réservoir (2) ; le dispositif se remplit comme une seringue avec aiguille.

Une fois la chambre distale remplie d'insuline Umuline® NPH, la seringue contenant le volume de peptide 1 est préparée à partir d'un flacon contenant la solution. Pour compenser les volumes morts du dispositif, 20µL s'ajoutent au volume de Peptide 1 à injecter soit un total de 170µL.

Il n'est pas nécessaire d'ajouter un surplus d'insuline car une fois injecté, le Peptide 1 expulse l'insuline qui aurait pu rester dans la chambre distale du réservoir. Le capuchon qui ferme la connexion proximale (1a) du piston creux (1) est retiré afin de connecter l'embout luer de la seringue contenant le Peptide 1. Le dispositif est alors prêt pour l'injection.

### Administration d'une solution d'insuline à 100UI/mL et d'une solution de peptide 1 à 200µg/mL via le dispositif selon l'invention :

Dans un premier temps la chambre distale est collapsée, et par la suite le Peptide 1 contenu dans la seringue est injecté à travers le canal interne (1b) en utilisant une procédure d'administration standard. La totalité des deux produits déchargés est collectée dans un flacon pour être analysée par HPLC.

### Résultats

Les données obtenues sont regroupées dans la Table 1 (comme indiqué ci-dessus, 20µL de la solution ont été ajoutés dans la seringue pour compenser les volumes morts)

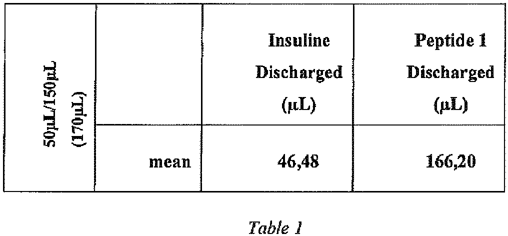

### Exemple 2 : Mise en évidence du fonctionnement des administrations réalisées avec un dispositif selon l'invention dans une seconde configuration

### Préparation d'une solution de Peptide 1 à 200µg/mL :

Approximativement 50mg de Peptide 1 sont pesés puis solubilisés dans 5mL d'acide acétique à 0,1% dont le poids est aussi déterminé par pesée. Par une dilution au 1/10 à partir de cette première solution à 2mg/mL est obtenue la solution finale à 200µg/mL.

### Solution d'insuline humaine lente (Umuline® NPH) à 100UI/mL :

Cette solution est une solution commerciale d'insuline humaine commercialisée par la société ELI LILLY sous le nom de Umuline® NPH. La solution est contenue dans un flacon commercial de 10mL à une concentration de 100UI/mL.

### Préparation du dispositif selon l'invention pour une solution d'insuline à 100UI/mL et une solution de peptide 1 à 200µg/mL à partir de deux seringues :

Cette configuration permet de remplir le réservoir par la connexion proximale (1a) à l'aide de deux seringues : la première contenant 20UI (20µL) d'insuline Umuline ® NPH et la seconde contenant 120 µL de Peptide 1. Le dispositif est équipé d'un bouchon luer butyle femelle standard (tel que représenté en (6) de la Figure 8) qui recouvre l'aiguille (4) et empêche le passage de liquides au travers.

La première seringue d'insuline Umuline® NPH est remplie à partir d'un flacon commercial de 10mL et est ensuite connectée par le cône luer mâle de la seringue insérée dans l'embout luer femelle de la connexion proximale (2) du piston creux (1).

Par cette connexion l'insuline Umuline® NPH est injectée dans la chambre distale du réservoir ; le dispositif étant obstrué à l'autre extrémité, le piston creux (1) se déplace au fur et à mesure que se remplit la chambre. Une fois déchargée la première seringue dans le dispositif selon l'invention, la seconde seringue est préparée de la même manière avec 120µL de Peptide 1, volume dans lequel 20µL constituent l'excédent de volume correspondant au volume mort du dispositif. Une fois la seconde seringue remplie de la dose de Peptide 1, elle vient substituer la première seringue, vide, dans l'embout luer de la connexion proximale (1a) du piston creux (1). Le capuchon qui recouvre l'aiguille est enlevé et le dispositif est prêt à injecter.

### Administration d'une solution d'insuline à 100UI/mL et une solution de peptide à 200µg/mL selon l'invention :

L'injection se réalise suivant le protocole décrit dans l'exemple 1.

### Exemple 3 : Mise en évidence du fonctionnement des administrations réalisées avec un dispositif selon l'invention dans une troisième configuration

### Solution de Glucagon Like Peptide-1 (GLP-1) analogue (Victoza®) à 6mg/mL :

Cette solution est une solution commerciale de Liraglutide commercialisée par la société NOVO NORDISK sous le nom de Victoza®. Ce produit est un analogue de GLP-1. La solution est contenue dans un stylo-injecteur Victoza® Pen pré-rempli et jetable, à une concentration de 6mg/mL.

Avant la première utilisation, le stylo-injecteur de Victoza® est amorcé comme décrit dans la notice d'utilisation pour s'assurer que le dispositif n'est pas défectueux.

Pour cela, on utilise une aiguille standard pour stylo-injecteur que l'on fixe sur le dispositif pour vérifier l'écoulement et cela avec une aiguille standard pour stylo-injecteur.

Le stylo-injecteur contenant la solution de Victoza® comporte une fenêtre permettant de voir la dose de produit sélectionnée, avec un marquage spécial indiquant la dose d'amorçage.

On tourne le sélecteur de dose jusqu'à cette marque et en appuyant sur le bouton d'injection on fait sortir une goutte de produit à travers l'aiguille. Si aucun produit n'apparaît, on répète l'opération 3 fois avant de changer d'aiguille et de recommencer tout le processus détaillé ci-dessus avec une aiguille neuve jusqu'à voir apparaître une goutte de produit. Cette opération de contrôle de l'écoulement se réalise à chaque changement de stylo-injecteur.

### Solution d'insuline humaine lente (Umuline® NPH) à 100UI/mL :

Cette solution est une solution commerciale d'insuline humaine commercialisée par la société ELI LILLY sous le nom de Umuline® NPH. Ce produit est une insuline à action intermédiaire. La solution est contenue dans un flacon commercial de 10mL à une concentration de 100UI/mL.

### Préparation du dispositif selon l'invention pour une solution de GLP-1 analogue à 6mg/mL et d'une solution d'insuline à 100UI/mL à partir d'un stylo-injecteur et d'une seringue :

Dans cette configuration, le dispositif selon l'invention est équipé d'un bouchon mâle luer standard (non représenté sur la figure 2, visible en (8) de la Figure 7) sur la connexion proximale (1a) et d'un adaptateur stylo-injecteur (11) qui se clippe ou se visse sur la connexion distale (2a) du corps réservoir (2) du dispositif. Sur cet adaptateur (11) un stylo-injecteur contenant l'analogue de GLP-1 Victoza® est vissé. Il est possible et recommandé de tenir le dispositif aiguille (4) tournée vers le haut (cachée dans l'adaptateur stylo-injecteur (11)). En vissant le stylo-injecteur, l'aiguille (4) de la connexion distale (2a) agit comme une connexion stylo-injecteur et perfore la membrane de la cartouche.

Une dose de 1,8mg (300µL) est sélectionnée sur le stylo-injecteur. Cette dose est intégralement transférée dans le réservoir à travers l'aiguille (4).

Tout en gardant le dispositif aiguille (4) tournée vers le haut, le stylo-injecteur est dévissé tout en laissant l'adaptateur stylo-injecteur (11) en place sur la partie distale du réservoir ; de cette manière l'aiguille (4) est protégée.

La seringue contenant 520µL d'insuline Umuline® NPH (volume dans lequel 20µL constituent l'excédent de volume correspondant au volume mort du dispositif) est préparée de manière standard à partir du flacon commercial de solution Umuline® NPH.

### Administration une solution de GLP-1 analogue à 6mg/mL et d'une solution d'insuline à 100UI/mL selon l'invention :

L'injection se réalise suivant le protocole décrit dans l'exemple 1.

### Exemple 4 : Mise en évidence du fonctionnement des administrations réalisées avec un dispositif selon l'invention dans une quatrième configuration

### Solution de Glucagon Like Peptide-1 (GLP-1) analogue (Victoza®) à 6mg/mL :

Cette solution est une solution commerciale de Liraglutide commercialisée par la société NOVO NORDISK sous le nom de Victoza®. Ce produit est un analogue de GLP-1. La solution est contenue dans un stylo-injecteur Victoza® Pen pré-rempli et jetable, à une concentration de 6mg/mL.

Avant la première utilisation, le stylo-injecteur de Victoza® est amorcé comme décrit dans la notice d'utilisation (vérification de l'écoulement), et cela avec une aiguille standard pour stylo-injecteur.

### Solution d'insuline analogue lente (Lantus®) à 100UI/mL :

Cette solution est une solution commerciale d'insuline Glargine commercialisée par la société SANOFI-AVENTIS sous le nom de Lantus®. Ce produit est une insuline analogue lente. La solution est contenue dans un stylo-injecteur SoloStar® pré-rempli et jetable, à une concentration de 100UI/mL.

Avant chaque utilisation, le stylo-injecteur SoloStar® contenant la solution de Lantus® est amorcé comme décrit dans la notice d'utilisation pour s'assurer que le dispositif n'est pas défectueux.

Pour cela, on utilise une aiguille standard pour stylo-injecteur que l'on fixe sur le dispositif pour vérifier l'écoulement. Le stylo-injecteur contenant la solution de Lantus® comporte une fenêtre permettant de voir la dose de produit sélectionnée. On tourne le sélecteur de dose jusqu'à la marque de 2UI d'insuline et en appuyant sur le bouton d'injection on fait sortir une goutte de produit à travers l'aiguille. Si aucun produit n'apparaît, on répète l'opération 3 fois avant de changer d'aiguille et de recommencer tout le processus détaillé ci-dessus avec une aiguille neuve jusqu'à voir apparaître une goutte de produit. Cette opération de contrôle de l'écoulement se réalise avant chaque utilisation du stylo injecteur de Lantus®.

### Préparation du dispositif selon l'invention pour une solution de GLP-1 analogue à 6mg/mL et d'une solution d'insuline à 100UI/mL à partir de deux stylo-injecteurs :

Dans cet exemple le dispositif selon l'invention est utilisé tel que représenté sur la Figure 6. L'adaptateur stylo-injecteur (11) est déjà mis en place sur la connexion distale (1a) du réservoir (2) du dispositif. Le stylo-injecteur contenant la solution de Victoza® est vissé sur le dispositif tenu aiguille d'administration (4) en l'air (cachée par l'adaptateur stylo-injecteur (11) c'est-à-dire stylo-injecteur en haut et dispositif connecteur-seringue en bas). En vissant, l'aiguille fixe (4) perfore la membrane de la cartouche contenant la solution de Victoza®.

Une dose de 0,6mg (soit 100µL) est sélectionnée sur le stylo-injecteur contenant la solution de Victoza®. Cette dose est intégralement transférée dans le réservoir à travers l'aiguille (4).

Tout en gardant le dispositif avec l'aiguille (4) tournée vers le haut, on dévisse le stylo-injecteur tout en laissant l'adaptateur stylo-injecteur (11) en place sur la partie distale du réservoir ; de cette manière l'aiguille (4) est protégée.

Le second stylo-injecteur contenant la solution d'insuline Lantus® se visse dans la connexion proximale (1a) du piston creux (1). Pour une dose à administrer de 15UI, une dose de 17UI est sélectionnée sur le stylo-injecteur pour tenir compte du volume mort du dispositif (2UI).

Une fois l'adaptateur stylo-injecteur (11) retiré pour dévoiler l'aiguille (4), le dispositif est prêt pour injection.

### Administration d'une solution de GLP-1 analogue à 6mg/mL et d'une solution d'insuline à 100UI/mL via le dispositif selon l'invention :

Une fois l'aiguille d'administration insérée au point d'injection, la chambre distale est collapsée à l'aide du piston creux (1) pour injecter la solution de Victoza® contenue dans le réservoir. Ce collapse est réalisé en appliquant une pression sur le dispositif en tenant le stylo-injecteur d'insuline par le corps mais sans activer le bouton de d'injection.

Ensuite, en appuyant sur le bouton d'injection du stylo-injecteur de Lantus® l'insuline est injectée et va occuper tous les volumes morts à l'intérieur du dispositif après son passage, expulsant le reste de la solution de Victoza®. Comme pour une injection habituelle avec un stylo-injecteur, on attend 10 secondes avec le doigt appuyé sur le bouton d'injection du stylo-injecteur de Lantus® pour s'assurer que toute la dose a bien été dispensée.

Une fois les deux produits injectés et le stylo-injecteur de Lantus® dévissé, le dispositif est jeté dans un container adapté.

### Exemple 5 : Mise en évidence du fonctionnement des administrations réalisées avec un dispositif selon l'invention dans une cinquième configuration

### Solution de Triptoréline (Décapeptyl®) à 0,1mg/mL:

Cette solution est une solution commerciale de Triptoréline commercialisée par la société IPSEN sous le nom de Décapeptyl®. Ce produit est un analogue de GnRH. Le produit se présente sous la forme d'un lyophilisat à reconstituer avec une ampoule de solvant.

Avant chaque injection, le lyophilisat doit être reconstitué avec 1mL de l'ampoule de solvant. Une fois la solution totalement claire et homogeneisée, elle est prête à l'emploi.

### Solution de follitropine bêta (Puregon®) à 300UI/0,36mL :

Cette solution est une solution commerciale de follitropine bêta commercialisée par la société MSD sous le nom de Puregon®. Ce produit est un analogue de l'hormone folliculo-stimulante (FSH). La solution est contenue dans un une cartouche de 0,36mL à utiliser avec le stylo-injecteur correspondant, le Puregon Pen®.

Avant sa première utilisation, le stylo doit être chargé avec la cartouche de Puregon® et être amorcé comme décrit dans la notice d'utilisation pour s'assurer que le dispositif n'est pas défectueux.

Pour cela, on utilise une aiguille standard pour stylo-injecteur fournie avec la cartouche de Puregon® que l'on fixe sur le dispositif pour vérifier l'écoulement. Le stylo-injecteur Puregon Pen® comporte une fenêtre permettant de voir la dose de produit sélectionnée. On tourne le sélecteur de dose jusqu'à la marque prévue à cet effet et en appuyant sur le bouton d'injection on fait sortir une goutte de produit à travers l'aiguille. Si aucun produit n'apparaît, on répète l'opération 3 fois avant de changer d'aiguille et de recommencer tout le processus détaillé ci-dessus avec une aiguille neuve jusqu'à voir apparaître une goutte de produit. Cette opération de contrôle de l'écoulement se réalise seulement avant la première utilisation du Puregon Pen®.

### Préparation du dispositif selon l'invention pour une solution de Follitropine bêta à 300UI/0,36 mL et d'une solution de Triptoreline à 0,1mg/mL à partir d'un styloinjecteur et d'une seringue :

Dans cette configuration, le dispositif selon l'invention est équipé d'un bouchon mâle luer standard (non représenté sur la figure 2, visible en (8) de la Figure 7) sur la connexion proximale (1a) et d'un adaptateur stylo-injecteur (11) qui se clippe ou se visse sur la connexion distale (2a) du corps réservoir (2) du dispositif. Sur cet adaptateur (11) un stylo-injecteur Puregon Pen® est vissé. Il est possible et recommandé de tenir le dispositif aiguille (4) tournée vers le haut (cachée dans l'adaptateur stylo-injecteur (11)). En vissant le stylo-injecteur, l'aiguille (4) de la connexion distale (2a) agit comme une connexion stylo-injecteur et perfore la membrane de la cartouche.

Une dose de 225UI (270µL) est sélectionnée sur le stylo-injecteur. Cette dose est intégralement transférée dans le réservoir à travers l'aiguille (4).

Tout en gardant le dispositif aiguille (4) tournée vers le haut, le stylo-injecteur est dévissé tout en laissant l'adaptateur stylo-injecteur (11) en place sur la partie distale du réservoir ; de cette manière l'aiguille (4) est protégée.

La seringue contenant 1020µL de Decapeptyl® (volume dans lequel 20µL constituent l'excédent de volume correspondant au volume mort du dispositif) est préparée de manière standard à partir du flacon commercial de solution de Decapeptyl® une fois reconstituée.

### Administration une solution de Follitropine bêta à 300UI/0,36 mL et d'une solution de Triptoreline à 0,1mg/mL selon l'invention :

L'injection se réalise suivant le protocole décrit dans l'exemple 1.

### Exemple 6 : Vérification de la non-contamination de la cartouche du stylo-injecteur lors de l'utilisation du dispositif selon l'invention

### Solution de Glucagon Like Peptide-1 (GLP-1) analogue (Victoza®) à 6mg/mL :

Cette solution est une solution commerciale de Liraglutide commercialisée par la société NOVO NORDISK sous le nom de Victoza. La solution est contenue dans un stylo-injecteur Victoza® Pen pré-rempli et jetable, à une concentration de 6mg/mL.

Avant la première utilisation, le stylo-injecteur de Victoza® est amorcé comme décrit ci-dessus à l'exemple 2 (vérification de l'écoulement), et cela avec une aiguille standard pour stylo-injecteur.

### Solution d'insuline analogue lente (Lantus®) à 100UI/mL :

Cette solution est une solution commerciale d'insuline Glargine commercialisée par la société SANOFI-AVENTIS sous le nom de Lantus®. Ce produit est une insuline analogue lente. La solution est contenue dans un stylo-injecteur SoloStar® pré-rempli et jetable, à une concentration de 100UI/mL.

### Evaluation de la non-contamination

Afin de vérifier l'action du piston plein (12) comme barrière physique empêchant toute contamination croisée entre les dispositifs ayant servi à charger les produits, une preuve de non-contamination est réalisée avec ces stylo-injecteurs. On détermine par analyse HPLC si le stylo-injecteur de Lantus® a été contaminé, ou contient des traces de GLP1 (Victoza®) qui était contenu dans le dispositif selon l'invention.

4UI (40µL) de la cartouche du stylo-injecteur de Lantus® utilisée dans l'exemple 4 sont déchargés dans un flacon pour analyse HPLC, complété à 1mL par une solution d'acide acétique à 0.1%. De la même manière un flacon de Victoza® est préparé. Après analyse HPLC les deux chromatogrammes sont comparés et l'absence de signal significatif correspondant au GLP1 (Victoza®) sur le chromatogramme de la solution d'insuline basale (Lantus®) est vérifiée.

La non-contamination de la cartouche contenue dans le stylo-injecteur de Lantus® après utilisation du dispositif selon l'invention est démontrée.

### Exemple 7 : Mise en évidence du fonctionnement et de la précision des administrations réalisées avec un dispositif selon l'invention dans une cinquième configuration

**Préparation du dispositif selon l'invention pour 3 solutions distinctes à partir de deux seringues er d'un stylo-injecteur :**

Pour réaliser cet exemple le dispositif tel que dans l'exemple 5 est utilisé, à la différence que la connexion proximale (1a) du piston creux (1) est de type luer.

### Etape 1 : Remplissage de la chambre distale (fluide A)

Cette étape se réalise comme pour le cas de 2 fluides dans l'Exemple 5.

### Etape 2 : Remplissage de la seconde chambre (fluide B)

Cette étape se réalise à partir d'une seringue préalablement remplie de la dose de produit B à injecter : tel que dans l'exemple 1, la seringue est connectée par la connexion proximale (1a) du piston creux (1).

La dose de produit B correspondant à la dose à administrer est ensuite injectée par le canal interne (1b) du piston creux (1) : le piston creux (1) étant plus mobile que le piston plein (12), il se déplace par rapport au réservoir, créant ainsi une deuxième chambre proximale remplie du fluide B. La dose de produit B à administrer ne doit pas dépasser un volume maximal (dépendant des dimensions du dispositif).

Une fois le contenu de la seringue B entièrement déchargé dans le dispositif, elle est déconnectée de la connexion proximale (1a) pour être remplacée par la seringue C.

### Etape 3 : Préparation de la seringue C (fluide C)

La préparation de la seringue C correspondant au troisième produit à décharger se réalise de manière standard ; toutefois, en tenant compte des volumes morts du dispositif, un léger excédent de produit est ajouté au volume à décharger. L'excédent de liquide dépend des dimensions du dispositif. Une fois la seringue de produit C placée dans l'embout luer du piston creux (1), le dispositif est prêt pour injection.

### Etape 4 : Injection

Pour réaliser l'injection, il est nécessaire de maintenir la tige de piston de la seringue C pour empêcher que le produit B contenu dans la seconde chambre distale formée entre le piston plein (12) et le piston creux (1) ne remonte dans la seringue C. Le produit A est déchargé en collapsant le piston plein (12), puis le produit B en collapsant le piston creux (1), et finalement le produit C à travers le canal interne (1b).

### Exemple 8 : Mise en évidence de l'amélioration de la surface de contact au point d'injection avec un dispositif selon l'invention versus un dispositif standard

Cet exemple a été mis au point pour mesurer l'impact et la douleur des injections lorsque l'utilisateur pousse l'aiguille travers la peau, plus particulièrement concernant l'impact du dispositif avec la peau en fin de pénétration de l'aiguille.
Le modèle élaboré donne une représentation visuelle de lésions créées par cet impact sur la surface fragile, telle qu'une feuille fine, de papier par exemple.
La surface fragile est posée à la surface d'un support spécifique pour simuler les injections (injection pad en anglais) et est maintenue en place et suffisamment tendue pour pouvoir pratiquer l'injection. Cette surface fragile sera marquée ou perforée pendant l'injection en fonction de l'appui et l'impact du dispositif en contact. La différence entre les marques laissées par les dispositifs testés permet de vérifier la commodité de leur contact avec la peau lors d'une injection standard.

Le pad équipé de la surface fragile est placé sous une machine Lloyd (REF) permettant de mesurer ou de programmer la force et la vitesse d'injection. Le déplacement à la même force et même vitesse est calculé sur une distance à partir du début de la pénétration du biseau de la canule jusqu'à une pression ou déformation de 5.00mm du dispositif d'injection dans le simulateur d'injection.

Avec une seringue classique équipée d'une aiguille standard, la perforation est visible sur un diamètre d'environ 2mm, accompagné d'une déformation ou des déchirures visibles sur un diamètre d'environ 1 cm sur la surface fragile.
Dans le cas du dispositif selon l'invention équipé de la même aiguille, une découpe centrale correspondant à la canule de l'aiguille entourée de la marque du cercle d'appui dudit dispositif sur la surface fragile. Aucune déchirure ni forte déformation n'est visible.

Ce modèle permet de confirmer que l'utilisation d'un dispositif selon l'invention est moins douloureuse pour le patient grâce à son prolongement permettant une meilleure répartition des forces d'injection sur la surface de la peau.

De manière à faciliter la compréhension des figures ci-jointes, la liste indiquée ci-après résume les composants desdites figures :

### Liste des composants :

1. Piston creux
   a) Connexion proximale
   b) Canal interne
2. Réservoir
   a. Connexion distale
   b. Paroi interne
   c. Fond
3. Joint
4. Aiguille fixe
5. Seringue A
6. Bouchon luer femelle standard
7. Seringue B
8. Bouchon luer mâle standard
9. Flacon
10. Septum Flacon
11. Adaptateur stylo injecteur
12. Piston plein
13. Stylo-injecteur A
14. Stylo-injecteur B
15. Aiguille standard
16. Clapets de sécurité
17. Rigole
18.
   a. Variation de surface de la paroi interne
   b. Variation de surface du piston plein
19. Contrôle de profondeur
20. Encoches de retenue
21. Crochets de sécurité

## Revendications

1. Dispositif du type connecteur-seringue pour administrer séparément au moins deux produits en quantités contrôlées par le biais d'une seule injection, ledit dispositif comprenant-un réservoir (2) et un piston creux (1) pouvant être déplacé à l'intérieur dudit réservoir (2) dans lequel :
- ledit piston creux (1) est ouvert par un canal interne (1b), de l'extrémité introduite à l'intérieur dudit réservoir (2) à l'autre extrémité dans une connexion proximale (1a)
- ledit piston creux (1) forme une barrière étanche avec la paroi interne (2b) dudit réservoir (2) et dans sa course fait varier le ou les volumes dudit réservoir (2) selon sa position d'une extrémité à l'autre à l'intérieur dudit réservoir (2) ; et
- ladite connexion proximale (1a) reste constamment accessible à l'extérieur dudit réservoir (2)
- ledit réservoir est ouvert à une extrémité dans une connexion distale (2a) et à l'autre extrémité de façon à introduire et laisser circuler ledit piston creux (1),
par distale s'entend la zone du dispositif la plus proche de la zone d'administration, par proximale s'entend la zone du dispositif la plus éloignée de la zone d'injection.

2. Dispositif selon la revendication 1, comprenant au moins un, de préférence un seul piston plein (12) pouvant se déplacer à l'intérieur dudit réservoir (2) **caractérisé en ce que**:
- ladite paroi interne (2b) présente à partir du fond (2c) dudit réservoir (2), de préférence sur une distance pratiquement égale à l'épaisseur dudit piston plein une variation de sa section destinée à rompre l'étanchéité entre ledit piston plein (12) et ladite paroi interne (2b) dudit réservoir (2), de préférence une augmentation de son diamètre (18a) et
- à l'exception de la distance sur laquelle ladite paroi interne (2b) présente une variation de sa section (18a), ledit piston plein (12) forme une barrière étanche avec ladite paroi interne (2b) dudit réservoir (2)
- lorsque ledit piston plein (12) se trouve au contact dudit fond (2c) dudit réservoir (2) il n'est plus étanche avec ladite paroi interne (2b) dudit réservoir (2) ; ce qui permet le passage desdits produits entre ledit piston plein (12) et ladite paroi interne (2b) dudit réservoir (2),

3. Dispositif selon la revendication 2 comprenant au moins un piston plein (12) **caractérisé en ce que** le au moins piston plein comporte sur sa face distale une variation de surface de préférence au moins une gorge ou une rainure (18b).

4. Dispositif selon l'une quelconque des revendications précédentes, **caractérisé en ce que** ledit connecteur-seringue peut être connecté à un ou plusieurs dispositifs d'administration du type seringue, stylo-injecteur, poche d'injection, flacon, cartouche, injecteur, pompe, aiguille, cathéter ou connecteur.

5. Dispositif selon l'une quelconque des revendications précédentes, **caractérisé en ce que** le diamètre dudit canal interne (1b) est le plus petit possible qui soit compatible avec le passage desdits produit à administrer et de préférence ledit diamètre est d'un diamètre supérieur ou au moins égal au diamètre du conduit de l'aiguille (4) lorsque la connexion distale (2a) est pourvue de ladite aiguille.

6. Dispositif selon l'une quelconque des revendications précédentes, **caractérisé en ce que** ledit connecteur-seringue comporte à ses extrémités proximale et/ou distale des connexions (1a) et (2a) qui peuvent être mâles ou femelles, et être choisies parmi les connections de type luer, luer-lock, vis, aiguille, septum, valve.

7. Dispositif selon l'une quelconque des revendications précédentes, **caractérisé en ce qu'**un adaptateur (11) qui permet la fixation d'un stylo-injecteur audit connecteur-seringue est fixé sur ladite connexion distale (2a) autour de son aiguille (4).

8. Dispositif selon l'une quelconque des revendications précédentes, **caractérisé en ce que** ledit connecteur-seringue est stérilisé avant remplissage est réalisé en matériaux compatibles avec les injections, **en ce que** ledit réservoir (2) est transparent et **en ce que** ledit piston creux (1) ou ledit réservoir (2) peuvent être gradués.

9. Dispositif selon l'une quelconque des revendications précédentes, **caractérisé en ce qu'**une partie de la connexion distale (2a) pourvue d'une aiguille est prolongée sur un diamètre comparable à celui dudit dispositif, jusqu'à la canule de ladite seule aiguille pour assurer un appui sur la peau au moment de l'injection.

10. Dispositif selon l'une quelconque des revendications précédentes, **caractérisé en ce que** ledit connecteur-seringue présente un mécanisme de blocage qui évite sa réutilisation et qui peut contrôler différentes profondeurs d'injection de ladite seule aiguille lorsque ledit piston creux (1) est introduit audit fond (2c) dudit réservoir (2).

11. Procédé de remplissage d'un dispositif connecteur-seringue défini à l'une quelconque des revendications précédentes, comprenant, à l'intérieur d'un corps externe dudit réservoir (2) un élément interne sous la forme dudit piston creux (1), fermant ledit corps réservoir d'un côté, procédé dans lequel les produits à conditionner sont introduits à l'intérieur dudit réservoir (2) du côté dudit connexion distale (2a) ou du côté dudit piston creux (1) avant ou après que ledit piston creux (1) ne soit introduit dans ledit réservoir (2).

12. Procédé de remplissage d'un dispositif connecteur-seringue selon la revendication 1, comprenant, à l'intérieur dudit réservoir (2) cloisonné dans ses volumes par un piston plein (12) et par ledit piston creux (1), procédé dans lequel les produits à conditionner sont introduits à l'intérieur dudit réservoir (2) du côté dudit
connexion distale (2a) après que ledit piston plein ait été positionné dans ledit réservoir ou du côté dudit piston creux (1), avant ou après que ledit piston plein (12) ou ledit piston creux (1) ne soit introduit dans ledit réservoir (2).

## Patentansprüche

1. Vorrichtung des Typs Spritzenverbinder zur getrennten Verabreichung von kontrollierten Mengen von mindestens zwei Produkten in einer einzigen Injektion, wobei die Vorrichtung umfasst:
- einen Behälter (2) und einen hohlen Kolben (1), der im Inneren des Behälters (2) verlagert werden kann, wobei
- der hohle Kolben (1) durch einen Innenkanal (1b) von dem in das Innere des Behälters (2) eingeführten Ende bis zum andere Ende in einer proximalen Verbindung (1a) offen ist;
- wobei der hohle Kolben (1) eine dichte Schranke mit der Innenwand (2b) des Behälters (2) bildet und bei seinem Hub das oder die Volumina des Behälters (2) entlang seiner Position von einem Ende zum anderen innerhalb des Behälters (2) variiert; und
- wobei die proximale Verbindung (1a) außerhalb des Behälters (2) ständig zugänglich bleibt;
- wobei der Behälter an einem Ende in einer distalen Verbindung (2a) und am anderen Ende offen ist, um den hohlen Kolben (1) einzuführen und umlaufen zu lassen,
wobei unter distal die Zone der Vorrichtung zu verstehen ist, die der Verabreichungszone am nächsten ist, und unter proximal die Zone der Vorrichtung zu verstehen ist, die am weitesten von der Injektionszone entfernt ist.

2. Vorrichtung nach Anspruch 1, umfassend mindestens einen, vorzugsweise nur einen vollen Kolben (12), der sich im Inneren des Behälters (2) verlagern kann, **dadurch gekennzeichnet, dass**:
- die Innenwand (2b) ausgehend vom Boden (2c) des Behälters (2), vorzugsweise über eine Distanz praktisch gleich der Dicke des vollen Kolbens, eine Variation ihres Querschnitts aufweist, die dazu bestimmt ist, die Abdichtung zwischen dem vollen Kolben (12) und der Innenwand (2b) des Behälters (2) zu durchbrechen, vorzugsweise eine Erhöhung ihres Durchmessers (18a), und
- mit Ausnahme der Distanz, über die die Innenwand (2b) eine Variation ihres Querschnitts (18a) aufweist, der volle Kolben (12) eine dichte Schranke mit der Innenwand (2b) des Behälters (2) bildet,
- wenn sich der volle Kolben (12) in Kontakt mit dem Boden (2c) des Behälters (2) befindet, dieser nicht mehr mit der Innenwand (2b) des Behälters (2) dicht ist; was den Durchgang der Produkte zwischen dem vollen Kolben (12) und der Innenwand (2b) des Behälters (2) ermöglicht.

3. Vorrichtung nach Anspruch 2, mindestens umfassend einen vollen Kolben (12), **dadurch gekennzeichnet, dass** der mindestens eine volle Kolben auf seiner distalen Fläche eine Oberflächenvariation, vorzugsweise eine Rille oder eine Nut (18b), umfasst.

4. Vorrichtung nach einem der vorhergehenden Ansprüche, **dadurch gekennzeichnet, dass** der Spritzenverbinder an eine oder mehrere Verabreichungsvorrichtungen des Typs Spritze, Injektionsstift, Injektionsbeutel, Flakon, Kartusche, Injektor, Pumpe, Nadel, Katheter oder Verbinder angeschlossen werden kann.

5. Vorrichtung nach einem der vorhergehenden Ansprüche, **dadurch gekennzeichnet, dass** der Durchmesser des Innenkanals (1b) möglichst der kleinste ist, der mit dem Durchgang der zu verabreichenden Produkte vereinbar ist, und dass vorzugsweise der Durchmesser ein Durchmesser größer oder mindestens gleich dem Durchmesser der Leitung der Nadel (4) ist, wenn die distale Verbindung (2a) mit der Nadel versehen ist.

6. Vorrichtung nach einem der vorhergehenden Ansprüche, **dadurch gekennzeichnet, dass** der Spritzenverbinder an seinen proximalen und/oder distalen Enden Verbindungen (1a) und (2a) umfasst, die Einsteck- oder Aufnahmeverbindungen sein und unter den Verbindungen des Typs Luer, Luer-Lock, Schraube, Nadel, Septum, Ventil ausgewählt sein können.

7. Vorrichtung nach einem der vorhergehenden Ansprüche, **dadurch gekennzeichnet, dass** ein Adapter (11), der die Befestigung eines Injektionsstiftes am Spritzenverbinder ermöglicht, auf der distalen Verbindung (2a) um ihre Nadel (4) herum befestigt ist.

8. Vorrichtung nach einem der vorhergehenden Ansprüche, **dadurch gekennzeichnet, dass** der Spritzenverbinder vor dem Befüllen sterilisiert wird und aus mit den Injektionen vereinbaren Materialien hergestellt ist, dass der Behälter (2) transparent ist, und dass der hohle Kolben (1) oder der Behälter (2) eine Gradeinteilung aufweisen können.

9. Vorrichtung nach einem der vorhergehenden Ansprüche, **dadurch gekennzeichnet, dass** ein Teil der distalen Verbindung (2a), der mit einer Nadel versehen ist, auf einem Durchmesser vergleichbar mit jenem der Vorrichtung bis zur Kanüle der einzigen Nadel verlängert ist, um zum Zeitpunkt der Injektion eine Anlage an der Haut zu gewährleisten.

10. Vorrichtung nach einem der vorhergehenden Ansprüche, **dadurch gekennzeichnet, dass** der Spritzenverbinder einen Blockiermechanismus aufweist, der seine Wiederverwendet verhindert und verschiedene Injektionstiefen der einzigen Nadel steuern kann, wenn der hohle Kolben (1) zum Boden (2c) des Behälters (2) eingeführt wird.

11. Verfahren zum Befüllen einer Spritzenverbindervorrichtung, die in einem der vorhergehenden Ansprüche definiert ist, umfassend innerhalb eines äußeren Körpers des Behälters (2) ein inneres Element in Form des hohlen Kolbens (1), der den Behälterkörper auf einer Seite verschließt, wobei bei dem Verfahren die zu verpackenden Produkte in das Innere des Behälters (2) auf der Seite der distalen Verbindung (2a) oder auf der Seite des hohlen Kolbens (1) eingeführt werden, bevor oder nachdem der hohle Kolben (1) in den Behälter (2) eingeführt wird.

12. Verfahren zum Befüllen einer Spritzenverbindervorrichtung nach Anspruch 1, umfassend im Inneren einen Behälter (2), der durch einen vollen Kolben (12) und durch den hohlen Kolben (1) in seine Volumina unterteilt ist, wobei bei dem Verfahren die zu verpackenden Produkte in das Innere des Behälters (2) auf der Seite der distalen Verbindung (2a) eingeführt werden, nachdem der volle Kolben in dem Behälter positioniert wurde, oder auf der Seite des hohlen Kolbens (1), bevor oder nachdem der volle Kolben (12) oder der hohle Kolben (1) in den Behälter (2) eingeführt wird.

## Claims

1. A syringe-connector type device for separately administering at least two products in controlled quantities by means of a single injection, said device comprising a reservoir (2) and a hollow plunger (1) that can be moved within said reservoir (2) wherein:
- said hollow plunger (1) is open by an internal channel (1b), from the end inserted inside said reservoir (2) to the other end in a proximal connection (1a)
- said hollow plunger (1) forms a sealed barrier with the inner wall (2b) of said reservoir (2) and in its stroke varies the volume or volumes of said reservoir (2) according to its position from one end to the other in the interior of said reservoir (2); and
- said proximal connection (1a) remains constantly accessible outside of said reservoir (2) and
- said reservoir is open at one end in a distal connection (2a) and at the other end so as to introduce and allow said hollow plunger (1) to circulate,
by distal it is meant the area of the device closest to the administration zone,
by proximal it is meant the area of the device furthest from the injection zone.

2. The device according to claim 1 comprising at least one, preferably a single solid plunger (12) movable within said reservoir (2) **characterized in that**:
- said inner wall (2b) has from bottom (2c) of said reservoir (2), preferably over a distance substantially equal to the thickness of said solid plunger, a variation of its section intended to break the sealing between said solid plunger (12) and said inner wall (2b) of said reservoir (2), preferably an increase in its diameter (18a) and
- with the exception of the distance on which said inner wall (2b) has a variation of its section (18a), said solid plunger (12) forms a sealed barrier with said inner wall (2b) of said reservoir (2)
- when said solid plunger (12) is in contact with said bottom (2c) of said reservoir (2) it is no longer sealed with said inner wall (2b) of said reservoir (2); which allows said products to pass between said solid plunger (12) and said inner wall (2b) of said reservoir (2).

3. The device according to claim 2 comprising at least one solid plunger (12), wherein the at least one solid plunger comprises on its distal face a surface variation preferably of at least one groove or one slot (18b).

4. The device according to any one of the preceding claims, **characterized in that** said syringe connector can be connected to one or more syringe-type administration devices, injector pen, injection bag, bottle, cartridge, injector, pump, needle, catheter or connector.

5. The device according to any one of the preceding claims, **characterized in that** the diameter of said internal channel (1b) is the smallest possible that is compatible with the passage of said product to be administered and preferably said diameter is greater in diameter than or at least equal to the diameter of the needle duct (4) when the distal connection (2a) is provided with said needle.

6. The device according to any one of the preceding claims, **characterized in that** said syringe-connector comprises at its proximal and/or distal ends connections (1a) and (2a) which can be male or female, and be chosen from the connections of luer, luer-lock, screw, needle, septum, valve type.

7. The device according to any one of the preceding claims, **characterized in that** an adapter (11) which allows the fixing of an injector pen to said syringe connector is fixed on said distal connection (2a) around its needle (4).

8. The device according to any one of the preceding claims, **characterized in that** said syringe connector is sterilized before filling is made of materials compatible with the injections, **in that** said reservoir (2) is transparent and **in that** said hollow plunger (1) or said reservoir (2) can be graduated.

9. The device according to any one of the preceding claims, **characterized in that** a portion of the distal connection (2a) provided with a needle is extended to a diameter comparable to that of said device, up to the cannula of said single needle to provide support on the skin at the time of injection.

10. The device according to any one of the preceding claims, **characterized in that** said syringe connector has a locking mechanism which avoids its reuse and which can control different injection depths of said single needle when said hollow plunger (1) is introduced to said bottom (2c) of said reservoir (2).

11. A Method for filling a syringe-connector device as defined in any one of the preceding claims, comprising, inside an external body of said reservoir (2), an inner element in the form of said hollow plunger (1), closing said reservoir body on one side, the method wherein the products to be packaged are introduced inside said reservoir (2) on the side of a distal connection (2a) or on the side of said hollow plunger (1) before or after said hollow plunger (1) is introduced into said reservoir (2).

12. Method for filling a syringe-connector device according to claim 1, comprising, inside said reservoir (2) partitioned in its volumes by a solid plunger (12) and by said hollow plunger (1), the method wherein the products to be conditioned are introduced inside said reservoir (2) on the side of a distal connection (2a) after said solid plunger has been positioned in said reservoir or on the side of said hollow plunger (1), before or after said solid plunger (12) or said hollow plunger (1) is introduced into said reservoir (2).
